# EUROPEAN PATENT SPECIFICATION

(11) **EP 4 121 003 B1**
(45) Date of publication and mention of the grant of the patent: **06.08.2025**
(21) Application number: 21712156.5
(22) Date of filing: 16.03.2021
(51) Int. Cl.: A61K 9/00, A61K 9/08, A61K 38/26, A61K 47/02, A61K 47/18, A61P 3/04, A61P 3/10

(54) **LIQUID FORMULATIONS OF GLUCAGON ANALOGUES**
FLÜSSIGE FORMULIERUNGEN VON GLUCAGON-ANALOGA
FORMULATIONS LIQUIDES D'ANALOGUES DE GLUCAGON

(30) Priority: 16.03.2020 EP 20163408
(43) Date of publication of application: 25.01.2023
(73) Proprietor: Zealand Pharma A/S, 2860 Søborg (DK)
(72) Inventor: VILLADSEN, Jesper Skodborg, 2860 Søborg (DK); GOTTSCHALK BØVING, Tine Elisabeth, 2860 Søborg (DK)
(74) Representative: Mewburn Ellis LLP
(86) International application number: PCT/EP2021/056651
(87) International publication number: WO 2021/185821

(56) References cited:
- WO-A1-2014/016300
- WO-A1-2019/008033
- WO-A1-2019/110838

## Description

### Field of the Invention

The present invention relates to formulations of glucagon analogues and their medical use, for example in the treatment of hypoglycaemia. In particular, the present invention relates to stable aqueous liquid formulations of glucagon analogues.

### Background of the Invention

Human preproglucagon is a 158 amino acid precursor polypeptide that is differentially processed in the tissues to form a number of structurally related proglucagon-derived peptides, including glucagon (Glu), glucagon-like peptide-1 (GLP-1), glucagon-like peptide-2 (GLP-2), and oxyntomodulin (OXM). These molecules are involved in a wide variety of physiological functions, including glucose homeostasis, insulin secretion, gastric emptying and intestinal growth, as well as regulation of food intake.

Native glucagon is a 29-amino acid peptide that corresponds to amino acids 53 to 81 of pre-proglucagon. Glucagon helps maintain the level of glucose in the blood by binding to glucagon receptors on hepatocytes, causing the liver to release glucose, stored in the form of glycogen, through glycogenolysis. As these stores become depleted, glucagon also stimulates the liver to synthesize additional glucose by gluconeogenesis. This glucose is released into the bloodstream, preventing the development of hypoglycaemia. This has led to the development of therapeutic formulations of glucagon or glucagon analogues which are intended for use in "rescue" situations for alleviating acute hypoglycaemia in diabetic subjects, for example to treat subjects who have received an excessively high dose of insulin. However, owing to the relatively low physical and chemical stability of native glucagon *per se,* the majority of glucagon products that are currently available commercially are provided in the form of freeze-dried, solid preparations that need to be reconstituted in an appropriate liquid medium immediately before use. As hypoglycaemic subjects may exhibit symptoms of dizziness and/or confusion, or in some cases may even become unconscious or semi-conscious, this means that they can be unable to carry out or complete the required initial liquid reconstitution and subsequent injection of the glucagon formulation in question. As a result, this reconstitution and injection may have to be performed by another person who is not experienced in processing the product in the limited time available before excessive glucagon aggregation occurs.

This has led to efforts in this field to produce formulations of glucagon analogues that, in addition to having satisfactorily high activity at the glucagon receptor, are sufficiently soluble (especially at physiological pH, where native glucagon is essentially insoluble at neutral pH) and physically and chemically stable in liquid formulations that are capable of immediate use without the need for reconstitution. These analogues (i) may advantageously be provided in the form of a ready-to-use liquid pharmaceutical formulation adapted for immediate injection, and (ii) may be able to be stored (including carried by the subject or patient in question under ambient conditions) for a sufficiently long period of time prior to use.

WO 2011/117417 (Novo Nordisk A/S) discloses modified glucagon peptides including a mutation at amino acid position 25 of glucagon 1-29 that are resistant to aggregation in aqueous solutions.

WO 2016/133863 (Eli Lilly and Company) describes powder compositions of glucagon for nasal administration that include a cyclodextrin and a phospholipid surfactant in a phase characterized by an XRPD mesopeak.

WO 2019/110838 (Adocia) relates to formulations of glucagon, co-polyamino acid bearing carboxylate fillers and hydrophobic radicals.

WO 2008/084237 (Arecor Limited) describes aqueous protein compositions in which a displacement buffer is used that has a pKa at least one unit greater than the pH at which the protein is stabilized.

WO 2014/016300 (Zealand Pharma A/S) discloses glucagon analogues suitable for use in liquid formulations, where the analogues have improved solubility and/or stability compared to native human glucagon. One of the glucagon analogues described in WO 2014/016300 is called dasiglucagon and is in clinical trials as a ready-to-use treatment for severe hypoglycaemia for the treatment of type 1 diabetes and for the treatment of congenital hyperinsulinism. The pharmacokinetic and pharmacodynamic characteristics of dasiglucagon and a comparison with GlucaGen^{®} is disclosed in Hövelmann et al, Diabetes Care, 2017 (https://doi.org/10.2337/dc17-1402).

WO 2017/053822 (Xeris Pharmaceuticals, Inc.) discloses liquid formulations of native glucagon peptide that use dimethyl sulfoxide (DMSO) as an aprotic polar solvent system. Ionization stabilizing excipients may also be added to improve solubilization. WO 2014/124151 (Xeris Pharmaceuticals, Inc.) discloses liquid formulations of glucagon peptide in DMSO that additionally include a cryoprotectant, such as trehalose, to protect the formulations against freeze-thaw cycles. However, the use of DMSO in the solvent system for these formulations has several significant disadvantages as it makes the viscosity of the formulations relatively high, which makes injection difficult, and leads to unwanted side effects, such as skin reactions, dry skin, headache, dizziness, drowsiness, nausea, vomiting, diarrhea, constipation, breathing problems, vision problems, blood problems and allergic reactions. DMSO also causes a garlic-like taste, breath and body odour. Further, DMSO dissolves plastics and rubber which may be a disadvantage when administration is performed by a delivery device, e.g. a injection pen, a syringe or a pump system. In some cases, the DMSO is used in combination with 15-20% of a co-solvent, such as propylene glycol or ethanol, to avoid the relatively high freezing temperature of formulations containing 80-100% DMSO. However, if more than 5% water is included in the formulations using DMSO and co-solvents, this decreases the chemical stability of the glucagon in the formulation.

It therefore remains a problem in this area to improve the formulation of glucagon analogues, in particular to provide stable liquid formulations that are capable of long term storage without undue levels of physical and chemical degradation of the active monomeric form of the peptide occurring and which overcome some of the disadvantages associated with existing formulations of glucagon or glucagon analogues.

### Summary of the Invention

Broadly, the present invention is based on studies reported in the examples that have investigated and identified excipients that are capable of use in aqueous liquid formulations of glucagon analogues that are suitable for long term storage as liquids, and are capable of use in single-dose (SD) or multi-dose (MD) formulations.

In these studies, the present inventors found that the buffering agent used in the formulations of the glucagon analogues had an important effect on the stability of aqueous formulations comprising glucagon analogues, in particular dasiglucagon. The experiments disclosed herein show that TRIS, ACES and MES buffers and/or acetate, citrate or succinate buffers, provide chemical stability to glucagon analogue formulations at mildly acidic to neutral pH as demonstrated by detection and quantification of degradation products. Comparative analysis of the degradation profiles show that these buffers provide increased stability relative to other tested buffer systems, such as histidine or phosphate, even where the formulations otherwise had the same pH, i.e. that the stability provided by the buffers is at least in part independent of their pH modulating properties. Importantly, in aspects of the present invention in which the formulations further comprise a preservative, such as meta-cresol, the formulations have long-term stability in a liquid form, and are therefore suitable for multi-dosing. This opens up the possibility of providing formulations of glucagon analogues in the form of a drug delivery device, and particularly single-dose or multi-dose delivery devices, such as a pre-filled syringe, an injector device, an injector pen, an adjustable dose auto-injector, a disposable auto-injector, a wearable injector, or an infusion pump, thereby providing patients with a ready-to-use formulation in a simpler, safer and more patient-friendly device.

The application describes a stable aqueous liquid pharmaceutical formulation comprising a glucagon analogue which is:
Hy-HSQGTFTSDYSKYLD-Aib-ARAEEFVKWLEST-OH
or a pharmaceutically acceptable salt and/or derivative thereof; wherein the formulation comprises:
   (a) the glucagon analogue, or the pharmaceutically acceptable salt and/or derivative thereof, present at a concentration of about 0.5 mg/mL to about 10 mg/mL,
   (b) TRIS, Bis-TRIS, ACES or MES present as a buffer at a concentration of about 25 mM to about 75 mM;
   (c) sodium chloride present as a tonicity modifier; and at a concentration of about 50 mM to about 600 mM; and
   (d) a pH of about 5.6 to about 7.0.

In a first aspect, the present invention provides a stable aqueous liquid pharmaceutical formulation comprising a glucagon analogue, which is:
Hy-HSQGTFTSDYSKYLD-Aib-ARAEEFVKWLEST-OH
or a pharmaceutically acceptable salt thereof;
wherein the formulation comprises:
   (a) the glucagon analogue present at a concentration of about 0.5 mg/mL to about 10 mg/mL;
   (b) TRIS, ACES or MES is present as a buffer at a concentration of about 25 mM to about 75 mM, and/or citrate, acetate or succinate is present as a buffer at a concentration of about 1 mM to about 30 mM;
   (c) sodium chloride present as a tonicity modifier and at a concentration of about 50 mM to about 600 mM; and
   (d) a pH of about 5.6 to about 7.0.

In some embodiments of a formulation comprising a glucagon analogue, or the pharmaceutically acceptable salt thereof, the formulation comprises the glucagon analogue, or pharmaceutically acceptable salt thereof present at a concentration of about 0.5 mg/mL to about 10 mg/mL, such as about about 0.5 mg/mL, about 0.6 mg/mL, about 0.7 mg/mL, about 0.8 mg/mL, about 0.9 mg/mL, or about 1.0 mg/mL.

In some aspects, the present inventors found that TRIS, ACES or MES buffers are capable of directly chemically stabilising the glucagon analogue independent of the pH of the formulation provided by the buffer. The fact that the direct chemical stabilisation is improved may be determined relative to stability of the glucagon analogue in a corresponding formulation in a phosphate buffer and/or a histidine buffer under the same test conditions.

In a further aspect, the present invention provides processes for producing the stable aqueous liquid pharmaceutical compositions of the present invention.

In a further aspect, the present invention provides a delivery device containing a stable aqueous liquid formulation comprising a glucagon analogue of the present invention. By way of example, the delivery device includes a pre-filled syringe, an injector device, an injector pen, an adjustable dose auto-injector, a disposable auto-injector, a wearable injector, or an infusion pump.

The application describes an article of manufacture or a kit comprising a container holding a stable aqueous liquid pharmaceutical composition of the invention.

The application describes a stable aqueous liquid formulation of the glucagon analogue of the present invention for use in therapy.

In a further aspect, the present invention provides a stable aqueous liquid formulation of the glucagon analogue of the present invention for use in the treatment of a disease or condition selected from hypoglycaemia (including, but not limited to, severe hypoglycaemia, acute hypoglycaemia, and chronic hypoglycaemia), type 2 diabetes, impaired glucose tolerance, type 1 diabetes, obesity, coronary heart disease, atherosclerosis, hypertension, dyslipidemia, hepatic steatosis, β-blocker poisoning, insulinoma or Von Gierkes disease. In particular, the formulation may be used for the treatment of forms of hypoglycaemia, for example where the hypoglycaemia is selected from the group consisting of diabetic hypoglycaemia, acute insulin-induced hypoglycaemia, hyperinsulinemic hypoglycaemia, non-diabetic hypoglycaemia, reactive hypoglycaemia, fasting hypoglycaemia, drug-induced hypoglycaemia, congenital hyperinsulinemic hypoglycaemia, alcohol-induced hypoglycaemia, gastric bypass-induced hypoglycaemia (including, but not limited to postprandial hypoglycaemia after Roux-en-Y gastric bypass), post bariatic hypoglycaemia or hypoglycaemia occurring during pregnancy. In some embodiments, the method comprises administering to a patient in need of treatment a therapeutically effective amount of a stable aqueous liquid formulation of the glucagon analogue of the present invention.

In some embodiments, severe hypoglycaemia is typified by very low blood sugar. In some embodiments, treatment of acute hypoglycaemia includes treating an acute episode of severe hypoglycaemia, including, but not limited to, an episode of severe hypoglycaemia typified by very low blood sugar.

The application describes a method of treating a patient having a disease or condition selected from hypoglycaemia (including, but not limited to, severe hypoglycaemia, acute hypoglycaemia, and chronic hypoglycaemia), type 2 diabetes, impaired glucose tolerance, type 1 diabetes, obesity, coronary heart disease, atherosclerosis, hypertension, dyslipidemia, hepatic steatosis, β-blocker poisoning, insulinoma or Von Gierkes disease. In particular, the formulation may be used for the treatment of forms of hypoglycaemia, for example where the hypoglycaemia is selected from the group consisting of: diabetic hypoglycaemia, acute insulin-induced hypoglycaemia, hyperinsulinemic hypoglycaemia, non-diabetic hypoglycaemia, reactive hypoglycaemia, fasting hypoglycaemia, drug-induced hypoglycaemia, congenital hyperinsulinemic hypoglycaemia, alcohol-induced hypoglycaemia, gastric bypass-induced hypoglycaemia, post bariatic hypoglycaemia (including, but not limited to postprandial hypoglycaemia after Roux-en-Y gastric bypass), or hypoglycaemia occurring during pregnancy, the method comprising administering to a patient in need of treatment a therapeutically effective amount of a stable aqueous liquid formulation of the glucagon analogue of the present invention.

The application describes the use of a stable aqueous liquid formulation of the glucagon analogue of the present invention in the manufacture of a medicament for the treatment of a disease or condition selected from hypoglycaemia(including, but not limited to, severe hypoglycaemia, acute hypoglycaemia, and chronic hypoglycaemia), type 2 diabetes, impaired glucose tolerance, type 1 diabetes, obesity, coronary heart disease, atherosclerosis, hypertension, dyslipidemia, hepatic steatosis, β-blocker poisoning, insulinoma or Von Gierkes disease. In particular, the formulation may be used for the treatment of forms of hypoglycaemia, for example where the hypoglycaemia is selected from the group consisting of: diabetic hypoglycaemia, acute insulin-induced hypoglycaemia, hyperinsulinemic hypoglycaemia, non-diabetic hypoglycaemia, reactive hypoglycaemia, fasting hypoglycaemia, drug-induced hypoglycaemia, congenital hyperinsulinemic hypoglycaemia, alcohol-induced hypoglycaemia, gastric bypass-induced hypoglycaemia (including, but not limited to postprandial hypoglycaemia after Roux-en-Y gastric bypass), post bariatic hypoglycaemia or hypoglycaemia occurring during pregnancy, the method comprising administering to a patient in need of treatment a therapeutically effective amount of a stable aqueous liquid formulation of the glucagon analogue of the present invention.

In some embodiments, a patient treated using a formulation according to the present invention is not in a state of starvation, does not have adrenal insufficiency, and/or does not have chronic hypoglycaemia. In some embodiments, a patient treated using a formulation according to the present invention is not experiencing decreased hepatic glycogen. In some embodiments, a patient treated using a formulation according to the disclosure does not have a tumour in his or her adrenal gland, including, but not limited to, a pheochromocytoma. In some embodiments, a patient treated using a formulation according to the present invention does not have a tumour in his or her pancreas, including, but not limited to, an insulinoma.

In some embodiments, a patient treated using a formulation according to the disclosure is six years of age or older.

In a further aspect, the present invention provides a process for producing a stable aqueous liquid pharmaceutical formulation comprising a glucagon analogue which is:
Hy-HSQGTFTSDYSKYLD-Aib-ARAEEFVKWLEST-OH
or a pharmaceutically acceptable salt thereof;
the process comprising formulating (a) the glucagon analogue, or the pharmaceutically acceptable salt thereof present at a concentration of about 0.5 mg/mL to about 10 mg/mL; (b) TRIS, ACES or MES present as a buffer at a concentration of about 25 mM to about 75 mM; (c) sodium chloride present as a tonicity modifier; and at a concentration of about 50 mM to about 600 mM; (d) a pH of about 5.6 to about 7.0; and optionally (e) meta-cresol at a concentration of about 1.0 mg/mL to about 5.0 mg/mL to produce the stable aqueous liquid pharmaceutical formulation. In preferred embodiments, the process provides a stable aqueous liquid pharmaceutical formulation that is capable of storage stable at 2-8°C for at least 6 months, at least 12 months, at least 18 months or at least 24 months.

In some embodiments, the glucagon analogue, or the pharmaceutically acceptable salt thereof is present at a concentration of about 0.5 mg/mL to about 10mg/mL such as about 0.5 mg/mL, about 0.6 mg/mL, about 0.7 mg/mL, about 0.8 mg/mL, about 0.9 mg/mL, or about 1.0 mg/mL.

The application describes the use of a stable aqueous liquid pharmaceutical formulation comprising a glucagon analogue which is:
Hy-HSQGTFTSDYSKYLD-Aib-ARAEEFVKWLEST-OH
or a pharmaceutically acceptable salt and/or derivative thereof;
for providing an aqueous liquid pharmaceutical formulation which is stable for 24 months when stored at 2-8°C, wherein the formulation comprises:
   (a) the glucagon analogue present at a concentration of about 0.5 mg/mL to about 10 mg/mL;
   (b) TRIS, ACES or MES present as a buffer at a concentration of about 25 mM to about 75 mM;
   (c) sodium chloride present as a tonicity modifier; and at a concentration of about 50 mM to about 600 mM;
   (d) a pH of about 5.6 to about 7.0; and optionally
   (e) meta-cresol at a concentration of about 1.0 mg/mL to about 5.0 mg/mL to produce the stable aqueous liquid pharmaceutical formulation.

In some embodiments, the glucagon analogue, or the pharmaceutically acceptable salt thereof is present at a concentration of about 0.5 mg/mL to about 10 mg/mL such as about 0.5 mg/mL, about 0.6 mg/mL, about 0.7 mg/mL, about 0.8 mg/mL, about 0.9 mg/mL, or about 1.0 mg/mL.

The application describes an aqueous liquid pharmaceutical formulation comprising a glucagon analogue that consist essentially of or consist of the glucagon analogue and the other components of the formulation as defined herein. Further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Detailed Description of the Invention

### Definitions

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration, However, D-configuration amino acids may also be present. Aib stands for α-aminoisobutyric acid.

### Glucagon Analogues

The term "native glucagon" refers to native human glucagon having the sequence H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH (SEQ ID NO: 1).

Preferred compounds used in the formulations of the present invention have at least glucagon agonist activity. This can be assessed in *in vitro* assays, for example as described in WO 2014/016300, in which production of cellular cyclic AMP (cAMP) is used to monitor the human glucagon receptor activity in the presence of glucagon analogues.

Among sequences disclosed herein are sequences incorporating an "Hy-" moiety at the amino terminus (N-terminus) of the sequence, and an "-OH" moiety moiety at the carboxy terminus (C-terminus) of the sequence. In such cases, and unless otherwise indicated, a "Hy-" moiety at the N-terminus of the sequence in question indicates a hydrogen atom, while an "-OH" moiety at the C-terminus of the sequence indicates a hydroxy group.

The glucagon analogues present in the formulations of the present invention have one or more amino acid substitutions, deletions, inversions, or additions compared with native glucagon. This definition also includes the synonym terms glucagon mimetics and/or glucagon agonists. Further, the analogues of the present invention may additionally have chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is C₁-C₄ alkyl. Furthermore, one or more side groups, or terminal groups, may be protected by protective groups known to the ordinarily-skilled peptide chemist. The α-carbon of an amino acid may be mono- or di-methylated.

The liquid formulations of the present invention employ a glucagon analogue represented by the formula:
Hy-HSQGTFTSDYSKYLD-Aib-ARAEEFVKWLEST-OH (SEQ ID NO: 2), Compound 1 or a pharmaceutically acceptable salt thereof.

In three letter amino acid code, this glucagon analogue (dasiglucagon) is represented by the formula:

H-His-Ser-Gln-Gly-Thr-Phe-Thr-Ser-Asp-Tyr-Ser-Lys-Tyr-Leu-Asp-Aib-Ala-Arg-Ala-Glu-
Glu-Phe-Val-Lys-Trp-Leu-Glu-Ser-Thr-OH

Compound 1 (SEQ ID NO: 2) demonstrates *in vitro* agonist activity at the human glucagon receptor, with EC₅₀ values of 0.0095 nM and 0.030 nM, respectively as reported in WO 2014/016300.

It should be understood that the peptides (drug substance) of the invention might also be provided in the form of a salt or other derivative. Salts include pharmaceutically acceptable salts, as described in the definitions.

Other derivatives of the glucagon analogues of the invention include coordination complexes with metal ions such as Mn²⁺ and Zn²⁺, esters such as *in vivo* hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed *in situ* releasing active compound or serve to inhibit clearance of the drug *in vivo.*

The glucagon analogues used in the formulations of the present invention can be synthesized using solid phase peptide synthesis techniques well known in the art. By way of example, this may be carried out on a CEM Liberty Peptide Synthesizer using Tentagel S PHB-Thr(tBu) resin (1.13 g, 0.24 mmol/g), COMU as coupling reagent, DMF as the solvent, and Fmoc-chemistry as described above. Pseudoprolines Fmoc-Phe-Thr (Ψ Me, Me pro)-OH (in position 6/7) and Fmoc-Glu-Ser (Ψ, Me, Me pro)-OH (in position 15/16) were used in the sequence. Once synthesized, the glucagon analogue may be cleaved from the resin, and the purification was performed on a Gemini-NX column (5 cm, C18, 10 micron) with a 35 ml/min flow of a mixture of buffer A (0.1% aqueous TFA) and buffer B (aqueous solution containing 0.1% TFA and 90% MeCN). Pooled fractions may be lyophilized and re-dissolved in water prior to further purification. The purity of the product may be determined by analytical HPLC and the monoisotopic mass determined by MS.

### Formulations of Glucagon Analogues

As described herein, the stable aqueous liquid pharmaceutical formulation according to the present invention comprise a glucagon analogue, a buffer, a tonicity modifier and are formulated to have a pH that is physiologically compatible with administration to a patient. In some embodiments, the formulations of the present invention further comprise a preservative, for example enabling multi-dose formulations to be produced. In the examples below, the present inventors determined that the choice of buffer used for formulating the compositions of the present invention had a decisive effect on their long term stability and that many commonly used buffers including phosphate buffer, histidine buffer, maleic acid and Milli-Q water (MQW) did not provide sufficient stability in an aqueous liquid formulation, for example for storage for 13, 26 or 52 weeks at 25°C. However, other types of buffer, notably TRIS, Bis-TRIS, ACES or MES buffers were capable of being used to formulate stable aqueous liquid formulations of the glucagon analogues. The experiments described herein also found that the stability provided by these buffers was capable of directly chemically stabilising the glucagon analogue, i.e. in a manner which was independent of the pH of the formulation provided by the buffer. This effect may be determined relative to a phosphate buffer and/or a histidine buffer under the same test conditions.

TRIS buffer refers to (tris(hydroxymethyl)aminomethane or 2-Amino-2-(hydroxymethyl)propane-1,3-diol) in IUPAC nomenclature.

Bis-TRIS buffer refers to (Bis-Tris methane or 2-[Bis(2-hydroxyethyl)amino]-2-(hydroxymethyl)propane-1,3-diol) in IUPAC nomenclature.

ACES buffer refers to (*N*-(2-Acetamido)-2-aminoethanesulfonic acid or 2-(carbamoylmethylamino)ethanesulfonic acid) in IUPAC nomenclature.

MES buffer refers to (2-(N-morpholino)ethanesulfonic acid or as 2-morpholin-4-ylethanesulfonic acid in IUPAC nomenclature.

Other buffers that can be used as alternatives or in addition to these buffers include citrate, acetate or succinate, for example in the form of sodium citrate, sodium acetate or sodium succinate. Preferably, these buffers are present at a concentration of about 1 mM to about 30 mM, more preferably about 10 mM to about 30 mM, and more preferably at a concentration of about 15 mM. The actual concentration used may take account of whether the buffers are used individually or in combination.

The experimental work leading to the present invention shows that stable formulations of glucagon analogues can avoid including a polar aprotic solvent in the liquid formulation, such as the use of dimethyl sulfoxide (DMSO) as used in WO 2017/053822 and WO 2014/124151 with the disadvantages that this brings to the formulations in use. Accordingly, preferably the formulations of the present invention substantially do not include aprotic polar solvent, and more preferably the formulations substantially do not include dimethyl sulfoxide (DMSO). In particular preferred embodiments, water is the sole solvent used to make the aqueous liquid formulation.

The work described herein also shows that contrary to the experience with formulations based on the use of DMSO, optionally in combination with co-solvents, it is possible to produce formulations in which water is present without substantially affecting the chemical or physical stability of the glucagon analogues in the formulations. Particularly useful embodiments of liquid pharmaceutical formulations of the invention are aqueous formulations, i.e. formulations comprising water. Such formulations may be in the form of an aqueous solution or an aqueous suspension. Preferred embodiments of aqueous pharmaceutical formulations of the invention are aqueous solutions. In the context of the invention the term "aqueous formulation" will normally refer to a formulation comprising at least 50 % by weight (50 % w/w) of water as a solvent, more preferably at least 75% w/w of water, more preferably at least 80% w/w of water, more preferably at least 85% w/w of water, more preferably at least 90% w/w of water, most preferably at least 95% w/w of water,. Likewise, the term "aqueous solution" will normally refer to a solution comprising at least 50 % w/w of water, and the term "aqueous suspension" to a suspension comprising at least 50 % w/w of water. In certain embodiments, aqueous formulations of the present invention substantially do not include aprotic polar solvents, such as dimethyl sulfoxide (DMSO). Substantially in this sense means that the aqueous formulations include less than 5% by volume (v/v) aprotic solvent, more preferably less than 2% by volume (v/v) aprotic solvent and even more preferably less that 1% by volume (v/v) aprotic solvent. Water is the sole solvent used to make the aqueous liquid formulations according to certain embodiments.

In some aspects of the present invention, the stable aqueous liquid formulations of a glucagon analogue, pharmaceutically acceptable salt and/or derivative thereof comprise:
(a) the glucagon analogue present at a concentration of about 0.5 mg/mL to about 10 mg/mL;
(b) TRIS, ACES or MES present as a buffer at a concentration of about 25 mM to about 75 mM;
(c) sodium chloride present as a tonicity modifier; and at a concentration of about 50 mM to about 600 mM; and
(d) a pH of about 5.6 to about 7.0.

In some embodiments of a formulation comprising a glucagon analogue, or the pharmaceutically acceptable salt thereof, the formulation comprises the glucagon analogue, pharmaceutically acceptable salt thereof or derivative thereof, present at a concentration of about 0.5 mg/mL to about 10 mg/mL, such as about 0.5 mg/mL, about 0.6 mg/mL, about 0.7 mg/mL, about 0.8 mg/mL, about 0.9 mg/mL, or about 1.0 mg/mL.

The term "pharmaceutically acceptable carrier" includes any of the standard pharmaceutical carriers or diluents, such as those used in compositions or formulations suitable for oral, pulmonary, rectal, nasal, topical, subcutaneous, intramuscular, intravenous, intraperitoneal, intradermal, transdermal or vaginal administration. Pharmaceutically acceptable carriers for therapeutic use are well known in the pharmaceutical art, and are described, for example, in Remington's Pharmaceutical Sciences, Mack Publishing Co. (A. R. Gennaro edit. 1985). Liquid formulations often employ unbuffered or buffered aqueous solutions as carriers. For example, sterile saline or phosphate-buffered saline (PBS) at slightly acidic, slightly alkaline or physiological pH may be used. Relevant pH-buffering agents include tris(hydroxymethyl)aminomethane (TRIS), 2-[Bis(2-hydroxyethyl)amino]-2-(hydroxymethyl)propane-1,3-diol (Bis-TRIS), 2-(carbamoylmethylamino)ethanesulfonic acid (ACES), maleic acid, 2-morpholin-4-ylethanesulfonic acid (MES), as well as mixtures thereof. The term further encompasses any agents listed in the US Pharmacopeia for use in animals or humans.

The term "pharmaceutically acceptable salt" in the context of the invention refers to a salt that is not harmful to the patient or subject to be treated therewith. Such salts are in general acid addition salts or basic salts. Acid addition salts include salts of inorganic acids and salts of organic acids. Non-limiting examples of suitable acid addition salts include hydrochloride salts, phosphate salts, formate salts, acetate salts, trifluoroacetate salts and citrate salts. Examples of basic salts include salts where the cation is selected from alkali metal ions, such as sodium and potassium, alkaline earth metal ions, such as calcium, as well as substituted ammonium ions, e.g. of the type NR(R') ₃⁺, where R and R' independently designate optionally substituted C₁₋₆alkyl, optionally substituted C₂₋₆alkenyl, optionally substituted aryl, or optionally substituted heteroaryl. Other examples of pharmaceutically acceptable salts are described in Remington's Pharmaceutical Sciences, 17th edition. Ed. Alfonso R. Gennaro (Ed.), Mack Publishing Company, Easton, PA, U.S.A., 1985 and more recent editions, and in the Encyclopaedia of Pharmaceutical Technology.

The term "treatment" (as well as "treating" and other grammatical variants thereof) as employed in the context of the invention refers to an approach for obtaining beneficial or desired clinical results. For the purposes of the present invention, beneficial or desired clinical results include, but are not limited to, alleviation of symptoms, diminishment of extent of disease, stabilization of (i.e. not worsening of) state of disease, delay or slowing of disease progression, amelioration or palliation of disease state, and remission (whether partial or total), whether detectable or undetectable. "Treatment" may also refer to prolongation of survival compared to expected survival in the absence of treatment. "Treatment" is an intervention performed with the intention of preventing the development of, or altering the pathology of, a disorder. Accordingly, "treatment" refers both to therapeutic treatment and to prophylactic or preventative measures. As used in the context of prophylactic or preventative measures, the pharmaceutical formulation need not completely prevent the development of the disease or disorder. Those in need of treatment include those already suffering from the disorder, as well as those in which development of the disorder is to be prevented. "Treatment" also means inhibition or reduction of an increase in pathology or symptoms (e.g. weight gain or hypoglycaemia) compared to the absence of treatment, and is not necessarily meant to imply complete cessation of the relevant condition.

A "stable" formulation is one in which the peptide therein essentially retains its physical stability and/or chemical stability and/or biological activity upon storage. Preferably, the formulation essentially retains its physical and chemical stability, as well as its biological activity upon storage. The storage period is generally selected based on the intended shelf-life of the formulation. The formulations of the present invention are provided as stable liquid formulations, e.g. stable aqueous liquid formulations. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993), for example. In the present invention, "stable" formulations include formulations in which at least 80%, more preferably at least 85%, more preferably at least 90%, more preferably at least 95%, more preferably at least 96%, more preferably at least 97%, more preferably at least 98%, and most preferably at least 99% of the glucagon analogue does not degrade in the formulation after it has been stored at 2-8°C for 18 months. It is also possible to test stability under accelerated condition which generally use an increased storage temperature in order to assess stability over reduced time periods. For example, storage at 25°C, sometimes referred to as under accelerated conditions, may be used to assess stability over a period of 13, 26, 39 or 52 weeks. By way of comparison, the use of other buffers such as phosphate buffer or histidine buffer leads to stability levels that are generally less than 80% after storage for a corresponding period.

Stability can be measured at a selected temperature for a selected time period, for example using elevated temperature to reduce the period over which a formulation is tested. Generally, storage at a temperature between 2 to 8°C denotes storage under normal refrigerated conditions. In certain embodiments, the formulation is stable under such conditions for at least 6 months, more preferably at least 12 months, more preferably at least 18 months, more preferably at least 24 months. Stability can be evaluated qualitatively and/or quantitatively in a variety of different ways, including evaluation of aggregate formation (e.g. using size exclusion chromatography, UV light scattering, dynamic light scattering, circular dichroism, by measuring turbidity, and/or by visual inspection); by assessing charge heterogeneity using cation exchange chromatography, image capillary isoelectric focusing (iclEF) or capillary zone electrophoresis; amino-terminal or carboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomeriation), clipping/hydrolysis/fragmentation (e.g. hinge region fragmentation), succinimide formation, unpaired cysteine(s), N-terminal extension, C-terminal processing, glycosylation differences, adduct formation etc.

A peptide "retains its physical stability" in a pharmaceutical formulation if it shows no sign (or very little sign) of aggregation, precipitation and/or denaturation upon e.g. visual examination of colour and/or clarity, or as measured by UV light scattering, dynamic light scattering, circular dichroism, or by size exclusion chromatography and is considered to still retain its biological activity.

A peptide "retains its chemical stability" in a pharmaceutical formulation, if the chemical stability at a given time is such that the peptide is considered to still retain its biological activity as defined below. Chemical stability can be assessed by detecting and quantifying chemically altered forms of the peptide. Chemical alteration may involve isomerization, oxidation, size modification (e.g. clipping) which can be evaluated using HPLC or size exclusion chromatography, SDS-PAGE and/or mass spectrometry, for example. Other types of chemical alteration include charge alteration (e.g. occurring as a result of deamidation) which can be evaluated by HPLC or ion-exchange chromatography or iclEF, for example.

As disclosed herein, the stable aqueous liquid formulations of a glucagon analogue, or a pharmaceutically acceptable salt thereof, of the present invention generally comprise:
(a) the glucagon analogue, or the pharmaceutically acceptable salt thereof, present at a concentration of about 0.5 mg/mL to about 10 mg/mL;
(b) TRIS, ACES or MES present as a buffer at a concentration of about 25 mM to about 75 mM;
(c) sodium chloride present as a tonicity modifier; and at a concentration of about 50 mM to about 200 mM; and
(d) a pH of about 5.6 to about 7.0.

In some embodiments of a formulation comprising a glucagon analogue, or the pharmaceutically acceptable salt and/or derivative thereof, the formulation comprises the glucagon analogue, pharmaceutically acceptable salt thereof present at a concentration of about 0.5 mg/mL to about 10 mg/mL , such as about about 0.5 mg/mL, about 0.6 mg/mL, about 0.7 mg/mL, about 0.8 mg/mL, about 0.9 mg/mL, or about 1.0 mg/mL.

By way of example, in some embodiments, the glucagon analogue is present at a concentration of about 1.0 mg/mL or about 4.0 mg/mL.

Preferably, in the stable aqueous liquid formulations of the present invention, the TRIS, or ACES is present as a buffer at a concentration of about 50 mM.

Preferably, in the stable aqueous liquid formulations of the present invention, the sodium chloride present as a tonicity modifier at a concentration of about 50 mM to about 600 mM, at a concentration of about 50 mM to about 500 mM, at a concentration of about 150 mM to about 200 mM or at a concentration of about 60 mM to about 120 mM.

Preferably, in the stable aqueous liquid formulations of the present invention, the formulation has a pH between about 5.8 and 6.7, or a pH between about 6.0 and 7.0, and more preferable a pH of about 6.0 or about 6.5. In some embodiments disclosed herein, single-dose (SD) formulation have a pH of about 6.5, while formulations intended for multi-dose use have a pH of about 6.0 or a pH of about 6.5.

Preferably, in the stable aqueous liquid formulations of the present invention, the formulation does not include an ionization stabilizing excipient selected from hydrochloric acid, nitric acid, sulphuric acid or a combination thereof.

Preferably, in the stable aqueous liquid formulations of the present invention, the formulation is a ready-to-use formulation.

Preferably, in the stable aqueous liquid formulations of the present invention, the formulation is stable at 2-8°C for at least 6 months, at least 12 months, at least 18 months or at least 24 months. Preferably, in the stable aqueous liquid formulations of the present invention, the glucagon analogue in the formulation retains at least about 90% of its biological activity after 18 months of storage 2-8°C. While storage at 2-8°C reflects storage under refrigerated conditions from production to end use, the liquid formulations of the present invention preferably have long term storage stability under ambient conditions, for example during the time between sale to the end user and administration.

Preferably, in the stable aqueous liquid formulations of the present invention are sterile.

Preferably, in the stable aqueous liquid formulations of the present invention, the formulation is administration to a subject by injection, for example via subcutaneous injection.

Preferably, in the stable aqueous liquid formulations of the present invention, the buffer is TRIS .

In some aspects, the stable aqueous liquid formulations of the present invention may further comprise a preservative. Preferably the preservative is meta-cresol, optionally at a concentration of about 1.0 mg/mL to about 5.0 mg/mL.

In one embodiment, the present invention provides a stable aqueous liquid formulation in which the glucagon analogue is present at a concentration of about 0.75 mg/mL to about 1.25 mg/mL, TRIS is present at a concentration of about 40 mM to about 60 mM, sodium chloride is present as a tonicity modifier at a concentration of about 150 mM to about 200 mM and the formulation has a pH of about 6.0 to about 7.0. In this aspect, preferably the stable aqueous liquid formulation comprises the glucagon analogue is present at a concentration of about 1.0 mg/mL, TRIS is present at a concentration of about 50 mM, sodium chloride is present as a tonicity modifier at a concentration of about 175 mM and the formulation has a pH of about 6.5. This formulation is generally envisaged as a single dose formulation.

In a further embodiment the present invention provides a stable aqueous liquid formulation in which the glucagon analogue is present at a concentration of about 3.0 to 5.0 mg/mL, TRIS is present at a concentration of about 40 mM to about 60 mM, sodium chloride is present at a tonicity modifier at a concentration of about 60 mM to about 120 mM, meta-cresol is present as a preservative at a concentration of about 3.0 mg/mL to about 4.0 mg/mL and the formulation has a pH of about 6.0 to about 7.0. In this aspect, preferably the glucagon analogue is present at a concentration of about 4 mg/mL, TRIS is present at a concentration of about 50 mM, sodium chloride is present as a tonicity modifier at a concentration of about 90 mM, meta-cresol is present as a preservative at a concentration of about 3.0 to about 4.0 mg/mL and the formulation has a pH of about 6.5. In this aspect, the formulation is generally envisaged as a multi-dose formulation, and therefore includes a preservative. An example of a multi-dose formulation has pH of about 6.5, 4.0 mg/mL of dasiglucagon, TRIS present at a concentration of about 50 mM TRIS, sodium chloride is present at a tonicity modifier at a concentration of about 90 mM and meta-cresol is present as a preservative at a concentration of about 3.15 mg/mL.

In a further embodiment the present invention provides a stable aqueous liquid formulation in which the glucagon analogue, or the pharmaceutically acceptable salt thereof, is present at a concentration of about 3.0 to 5.0 mg/mL, TRIS, or MES is present as a buffer at a concentration of about 25 mM to about 75 mM, and/or citrate, acetate or succinate is present as a buffer at a concentration of about 1 mM to about 30 mM, sodium chloride is present at a tonicity modifier at a concentration of about 50 mM to about 150 mM, meta-cresol is present as a preservative at a concentration of about 3.0 mg/mL to about 4.0 mg/mL and the formulation has a pH of about 6.0 to 7.0. In this aspect, preferably the glucagon analogue, or the pharmaceutically acceptable salt thereof, is present at a concentration of about 4 mg/mL, TRIS, ACES, MES buffer is present at a concentration of about 50 mM or citrate, acetate and/or succinate buffer is present at a concentration of about 15 mM, sodium chloride is present at a tonicity modifier at a concentration of about 90 mM, meta-cresol is present as a preservative at a concentration of about 3.0 to about 4.0 mg/mL and the formulation has a pH of about 6.5.

The term "stabilized formulation" as employed herein refers to a formulation having increased physical stability, increased chemical stability or increased physical and chemical stability.

The term "physical stability" as used herein refers to a measure of the tendency of a peptide (e.g., a compound of the invention) to form soluble or insoluble aggregates of the peptide as a result of exposure of the peptide to stresses and/or interaction with interfaces and surfaces that are destabilizing, such as hydrophobic surfaces and interfaces. Physical stability of aqueous peptide formulations may be evaluated by means of visual inspection and/or turbidity measurements after exposing the formulation, filled in suitable containers (e.g. cartridges or vials), to mechanical/physical stress (e.g. agitation) at different temperatures for various time periods. A formulation may be classified as physically unstable with respect to peptide aggregation when it exhibits visual turbidity. Alternatively, the turbidity of a formulation can be evaluated by simple turbidity measurements well-known to the skilled person.

Physical stability of an aqueous peptide formulation can also be evaluated by using an agent that functions as a spectroscopic probe of the conformational status of the peptide. The probe is preferably a small molecule that preferentially binds to a non-native conformer of the peptide. One example of such a small-molecular spectroscopic probe is Thioflavin T, which is a fluorescent dye that has been widely used for the detection of amyloid fibrils. In the presence of fibrils, and perhaps also other peptide configurations, Thioflavin T gives rise to a new excitation maximum at about 450 nm and enhanced emission at about 482 nm when bound to a fibril form of a peptide. Unbound Thioflavin T is essentially non-fluorescent at the wavelengths in question.

The term "chemical stability" as used herein refers to stability of a peptide with respect to covalent/chiral chemical changes in the peptide structure that lead to formation of chemical degradation products with potentially lower biological potency and/or potentially increased immunogenicity compared to the native peptide structure. Various chemical degradation products can be formed, depending on the type and detailed nature of the native peptide and the environment to which the peptide is exposed. In practice, elimination of chemical degradation in peptide formulations in general cannot be avoided completely, and the formation of increasing amounts of chemical degradation products is often seen during storage and use of such formulations, as is well-known to the person skilled in the art. Many peptides are susceptible to a degradation process in which the side-chain amide group in glutaminyl or asparaginyl residues is hydrolysed to form a free carboxylic acid. Other degradation pathways involve formation of high-molecular-weight transformation products in which two or more peptide molecules become covalently bound to each other through transamidation and/or disulfide interactions, leading to formation of covalently bound oligomer and polymer degradation products (see, e.g., Stability of Protein Pharmaceuticals, Ahern. T.J. and Manning M.C., Plenum Press, New York 1992). Furthermore, hydrolysis of the peptide back bone leading to peptide fragments is well known. Oxidation (e.g., of methionine residues) is another form of chemical degradation of peptides. The chemical stability of a peptide formulation may be evaluated by measuring the amounts of chemical degradation products at various time-points after exposure to different environmental conditions (for example, formation of degradation products may often be accelerated by increasing temperature). The amount of each individual degradation product may be determined by separation of the degradation products to generate a "degradation profile". The separation is usually done on the basis of molecular size and/or charge using various chromatographic techniques (e.g. SEC-HPLC and/or RP-HPLC). The oligomers may be covalent oligomers or non-covalent oligomers. A covalent oligomer is that two or more molecules are linked through a covalent chemical bond and then bond is generally irreversible. In contrast, in non-covalent oligomers one or more molecules are linked together through an interaction that is not a covalent chemical bond, for example an ionic interaction, a hydrophobic interaction, etc. While in some cases this may be reversible, generally the interactions are so strong that they are practically irreversible and, as with covalent oligomers result in active drug substance not being pharmaceutically available.

The chemical instability of glucagon *per se* at low pH is mainly due to isomerisation and cleavage of aspartic acid residues, deamidation of glutamine residues and oxidation of methionine. Generally speaking, Asn and Gln deamidation occurs at high pH, with significant rates at physiological pH around pH 7.4 via a cyclic imide ring intermediate which can open to create L-Asp and L-isoAsp or L-Glu and L-isoGlu, respectively. The cyclic imide ring intermediate also may lead to the formation of small amounts of the corresponding D-isomers, indicating a slow racemisation of the cyclic imide.

At pH values below physiological pH, the rate of deamidation of Asn and Gln is reduced, but the rate of formation of a cyclic imide from Asp and Glu, and hence isomerisation, increases with decreasing pH. Cyclic imide formation is greatest between pH 4 and pH 6. Formation of the cyclic imide intermediate can also result in cleavage of the peptide sequence.

As outlined above, a "stabilized formulation" may thus refer to a formulation with increased physical stability, or increased chemical stability, or increased physical and chemical stability. In general, a formulation should be stable during use and storage (in compliance with recommended use and storage conditions) at least until the specified expiration date is reached.

In certain embodiments of liquid pharmaceutical formulations of the invention, the formulation is stable for at least 2 weeks of usage and for at least 6 months of storage. In further embodiments, the formulation is stable for at least 2 weeks of usage and for at least one year of storage. In still further embodiments, the formulation is stable for at least 2 weeks of usage and for at least two years of storage. In other embodiments, the formulation is stable for at least 4 weeks of usage and for at least two years of storage, or even for at least 4 weeks of usage and for more than 3 years of storage. Particularly useful embodiments of such pharmaceutical formulations of the invention are stable for at least 6 weeks of usage and for at least 3 years of storage. In this regard, the term "usage" for the purposes of this paragraph refers to taking the pharmaceutical formulation out of storage for the purpose of employing the formulation for therapeutic purposes, and thereby subjecting it to varying ambient conditions (conditions of light, dark, temperature, agitation etc.), whilst the term "storage" for the purposes of this paragraph refers to storage under non-agitated conditions in a refrigerator or freezer at a temperature not exceeding about 5°C. The skilled worker will understand the typical range of usage and storage conditions that these pharmaceutical formulations may be subjected to.

As described herein, the stable aqueous liquid formulations of the glucagon analogues of the present invention include a buffer to provide the pH and a tonicity modifier. In accordance with normal pharmaceutical practice, the formulations of the present invention are sterile and/or free from reducing agent. The liquid formulations of the present invention are aqueous, liquid formulations.

The term "buffer" as used herein denotes a pharmaceutically acceptable agent which stabilizes the pH of a pharmaceutical formulation. Suitable buffers are well known in the art and can be found in the literature. In preferred embodiments, the buffer is selected from the group consisting of a TRIS buffer, ACES buffer or MES buffer and/or citrate, acetate or succinate buffers, as these buffers provided stable formulations in which the glucagon analogues dissolved and did not become viscous, cloudy or precipitate the peptide drug. In preferred embodiments, the buffer is TRIS. Generally, the buffer will be present at a concentration of about 25 mM to about 75 mM. In certain embodiments, the buffer will be present at a concentration of about 50 mM. In certain embodiments, the TRIS, ACES or MES buffer provide direct chemical stabilisation of the glucagon analogue independent of the pH of the formulation. In other embodiments, the TRIS, ACES or MES buffer provides improved direct chemical stabilisation as determined relative to a phosphate buffer and/or histidine buffer under the same test conditions. In other embodiments, in particular those relating to multi-dose formulations in which a preservative such as metal cresol is present, the buffers also include the use of citrate, acetate or succinate at a concentration of about 1 mM to about 30 mM. In some embodiments, the formulation provides improved chemical stabilisation relative to a formulation in which the TRIS, ACES, or MES buffer is replaced by a phosphate buffer and/or a histidine buffer of the same concentration and pH evaluated under the same test conditions. For example, the formulation may have a degradation profile following 52 weeks storage at 25°C after which the formulation contains one or more of less than 5% of Pyro-Glu 4-29, less than 7% of Trp/Tyr oxidation, less than 4% of Kynurenine, less than 5% of F-4-29 + F5-29 and/or less than 2% of F3.29 after storage for 52 weeks at 25°C, wherein all percentages are determined by HPLC.

The term "tonicity modifier" as used herein denotes pharmaceutically acceptable tonicity agents that are used to modulate the tonicity of the formulation. The formulations of the present invention are preferably isosmotic, that is they have an osmotic pressure that is substantially the same as human blood serum. The tonicity modifier used in the formulations is preferably sodium chloride. The concentration of the tonicity modifier will be dependent on the concentration of other components of the formulation, especially where the formulation is intended to be isosmotic. Typically, sodium chloride will be employed as a tonicity modifier at a concentration of about 50 mM to 600 mM. In certain embodiments, the concentration of sodium chloride present as a tonicity modifier in the present formulations may be from about 50 mM to about 125 mM, about 75 mM to about 120 mM, about 90 mM to about 115 mM, or about 110 mM. In certain embodiments, the concentration of sodium chloride present as a tonicity modifier in the present formulations may be from about 150 mM to about 200 mM, optionally about 175 mM. In a preferred embodiment, sodium chloride may be present as a tonicity modifier in formulations comprising a preservative at concentrations of about 50 mM to about 150 mM, and most preferably at a concentration of about 90 mM, or about 75 mM or about 125 mM.

Generally, the components and amounts of the liquid formulations of the present invention are chosen to provide a formulation with a pH of about 5.6 or a pH of about 6.0 to about 7.0. In a preferred embodiment, the formulations may have a pH between about 6.2 and about 6.8. More preferably the formulations may have a pH of between about 6.3 and about 6.7. More preferably the formulations may have a pH of between about 6.4 and about 6.6. Alternatively or additionally, the formulations may have a pH of about 6.5 for both single dose and multi-dose formulations.

In certain embodiments, the formulations of the present invention further comprise a preservative. In preferred embodiments, the preservative is meta-cresol, present at a concentration of about 1.0 mg/mL to about 5.0 mg/mL, more preferably of about 2.0 mg/mL to about 4.0 mg/mL and most preferably at a concentration of about 3.0 mg/mL to about 4.0 mg/mL. The amount of preservative depends on the need to be able to effectively kill a range of bacterial types as needed for regulatory approval.

The formulations of the present invention have a degradation profile determined by chromatographic separation (e.g. by size-exclusion chromatography (SEC) and/or reverse-phase chromatography (RPC)). In particular, a further finding of the experiments described herein is that the profile of the degradation products of buffers that produced stable formulations according to the present invention were very similar after storage for 52 weeks at 25°C, while the degradation product profiles of buffers that did not produce stable formulations was different. In the following table the % of the degradation products was measured using HPLC.

Pyro-Glu(4-29) is a product where the backbone nitrogen of Gln in position 4 reacts with the side chain amide and forms an internal lactam ring. Subsequent hydrolysis of the amide bond between Ser in position 3 and the lactamized Glu moeity results in a degradation product where the first three amino acids have been cleaved off and there is pyro-Glu moiety at the N-terminus. Oxidation of Tyr/Trp leads to the incorporation of a hydroxyl group in the aromatic rings of Tyr and Trp. Kynurenine is a well known oxidation product of Trp residues. The addition of succinic acid relates to addition of dasiglucagon to the double bond in maleic acid buffer. FX¹-X² is a fragment containing amino acids X¹ to X².

| **Buffer** | **Major degradation product and comparison to TRIS, Bis-TRIS and MES** | **Degradation pathway** |
|---|---|---|
| Phosphate | RT 1.35, Pyro-Glu(4-29): 7.5% vs 0.4% | Hydrolysis |
| Histidine | RRT 0.22, Oxidation of Tyr/Trp, +16Da, 9.9% vs 0.3% | Oxidation |
| | RRT 0.75, Kynurenine, +4Da: 4.7% vs 0.2% | |
| Maleic acid | RRT 1.02, Addition of succinic acid, +116Da: 12.5% vs 0.0% | Addition of succinic acid (addition to the double bond in maleic aid) Hydrolysis |
| | RRT 1.23, F-4-29 + F5-29: 6.3% vs 0.6% | |
| | RRT1.13, F-3.29: 2.6% vs 0.2% | |

In summary, TRIS, Bis-TRIS, MES and ACES protects dasiglucagon from hydrolysis and oxidation compared to phosphate, histidine and maleic acid and there is no significant reactions between TRIS, Bis-TRIS, MES and dasiglucagon. A small amount (0.9% after 52 weeks at 25°C) of dasiglucagon condensed with ACES (+165 Da) is formed over 52 weeks).

Accordingly, in some aspects, the formulations of the present invention will have less than 5% of Pyro-Glu 4-29 after storage for 52 weeks at 25°C as determined by HPLC, more preferably less than 4%, more preferably less than 3%, more preferably less than 2%, more preferably less than 1% and most preferably less than 0.5%.

Accordingly, in some cases, the formulations of the present invention will have less than 7% of Trp/Tyr oxidation after storage for 52 weeks at 25°C as determined by HPLC. more preferably less than more preferably less than 5%, more preferably less than 4%, more preferably less than 3%, more preferably less than 2%, more preferably less than 1% and most preferably less than 0.5%.

Accordingly, in some cases, the formulations of the present invention will have less than 4% of Kynurenine after storage for 52 weeks at 25°C as determined by HPLC; more preferably less than 3%, more preferably less than 2%, more preferably less than 1% and most preferably less than 0.5%.

Accordingly, in some cases, the formulations of the present invention will have less than 5% of F-4-29 + F5-29 after storage for 52 weeks at 25°C as determined by HPLC, more preferably less than 4%, more preferably less than 3%, more preferably less than 2%, more preferably less than 1% and most preferably less than 0.5%.

Accordingly, in some cases, the formulations of the present invention will have less than 2% of F3.29 after storage for 52 weeks at 25°C as determined by HPLC, more preferably less than 1% and most preferably less than 0.5%.

In one aspect, the stable aqueous liquid formulation comprises the glucagon analogue at a concentration of about 0.75 mg/mL to about 1.25 mg/mL, TRIS at a concentration of about 40 mM to about 60 mM, sodium chloride as a tonicity modifier at a concentration of about 150mM to about 200 mM and the formulation has a pH of about 6.2 to about 6.8.

In another aspect, the stable aqueous liquid formulation comprises the glucagon analogue at a concentration of about 3.0 to 5.0 mg/mL, TRIS at a concentration of about 40 mM to about 60 mM, sodium chloride as a tonicity modifier at a concentration of about 60 mM to about 120 mM, meta-cresol as a preservative at a concentration of about 3.0 mg/mL to about 4.0 mg/mL and the formulation has a pH of about 6.0.

In certain embodiments, the stable liquid formulation of the glucagon analogues is a ready-to-use formulation. The term "ready-to-use" as used herein, refers to a formulation that does not require constitution or dilution with a prescribed amount of diluent, e.g., water for injection or other suitable diluent, before use by the designated route of administration.

### Medical Conditions

The glucagon analogue pharmaceutical formulations of the present invention may be useful in the treatment or prevention of a variety of conditions or disorders. Optionally, the formulations may be used in combination with one or more additional therapeutically active substances. Relevant therapeutic uses thus include: treatment or prevention of hypoglycaemia (including, but not limited to acute hypoglycaemia, chronic hypoglycaemia or severe hypoglycaemia), type 2 diabetes (including disease progression in type 2 diabetes), impaired glucose tolerance, type 1 diabetes, obesity (including diseases or states related to overweight or obesity), coronary heart disease, atherosclerosis, hypertension, dyslipidemia, hepatic steatosis, β-blocker poisoning, insulinoma and Von Gierkes disease; preventing a subject from becoming overweight; reducing body weight; decreasing food intake; increasing energy expenditure; delaying progression from impaired glucose tolerance (IGT) to type 2 diabetes; delaying progression from type 2 diabetes to insulin-requiring diabetes; regulating appetite or inducing satiety (including treatment of bulimia and treatment of binge-eating); and preventing weight regain after successful weight loss. As a general principle, pharmaceutical formulations of the invention may be useful to control blood glucose levels.

Among forms of hypoglycaemia capable of treatment or prevention in accordance with the invention are diabetic hypoglycaemia, acute insulin-induced hypoglycaemia, hyperinsulinemic hypoglycaemia, severe hypoglycaemia, non-diabetic hypoglycaemia, reactive hypoglycaemia, fasting hypoglycaemia, drug-induced hypoglycaemia, congenital hyperinsulinemic hypoglycaemia, alcohol-induced hypoglycaemia, gastric bypass-induced hypoglycaemia (including, but not limited to postprandial hypoglycaemia after Roux-en-Y gastric bypass), post bariatic hypoglycaemia or hypoglycaemia occurring during pregnancy.

In a further embodiment, the liquid formulation comprising a glucagon analogue of the present invention is contained in a delivery device. In some embodiments, the delivery device is a pre-filled syringe, an injector device, an injector pen, an adjustable dose auto-injector, a disposable auto-injector, a wearable injector, or an infusion pump.

Formulations of the invention may be administered in various dosage forms, for example solutions, suspensions or emulsions, and are useful in the formulation of controlled-, sustained-, protracted-, retarded- or slow-release drug delivery systems. More specifically, but not exclusively, pharmaceutical formulations of the invention are useful in connection with parenteral controlled-release and sustained-release systems, well known to those skilled in the art. General reference may be made in this connection to Handbook of Pharmaceutical Controlled Release (Wise, D.L., ed., Marcel Dekker, New York, 2000) and Drugs and the Pharmaceutical Sciences vol. 99: Protein Formulation and Delivery (MacNally, E.J., ed., Marcel Dekker, New York, 2000).

Parenteral administration (of a liquid pharmaceutical formulation of the invention) may be performed, for example, by subcutaneous, intramuscular, intraperitoneal or intravenous injection using a syringe, such as a pen-like syringe and/or a prefilled syringe.

Parenteral administration (of a liquid pharmaceutical formulation of the invention) may be performed, for example, by subcutaneous, intramuscular, intraperitoneal or intravenous injection using an autoinjector, such as an autoinjector comprising a syringe. An autoinjector may be single-use or multi-use and may provide (and/or be configured to provide) one or more doses of the liquid pharmaceutical formulation. In some embodiments, parenteral administration is performed by injecting a formulation of the disclosure into the lower abdomen, buttocks, thigh, or upper arm (including, but not limited to, the outer upper arm) of the patient. Alternatively, parenteral administration can take place using an infusion pump, e.g. in the form of a device or system borne by a subject or patient and comprising a reservoir containing a liquid formulation of the invention and an infusion pump for delivery/administration of the formulation to the subject or patient, or in the form of a corresponding miniaturized device suitable for implantation within the body of the subject or patient.

In some cases, a patient may be treated by administering a single dose of formulation to the patient or by administering multiple doses to the patient. In some cases, a patient may be administered a first dose of formulation and, subsequently, be administered a second dose of formulation. In some cases, a patient may be administered a first dose of formulation and, subsequently, be administered a second dose of formulation and, subsequently, be administered one or more further doses of formulation (e.g., a third dose and, possibly, a fourth or further dose).

### Examples

The following examples are provided to illustrate preferred aspects of the invention and are not intended to limit the scope of the invention.

### Example 1

### Materials and Methods

### Synthesis of Dasiglucagon

Dasiglucagon was synthesized by Bachem AG. The Dasiglucagon formulations were prepared and analysed by Zealand Pharma, A/S, Denmark. Alternatively, the glucagon analogues may be synthesized as described in WO 2014/016300 (Zealand Pharma A/S)

### Chemical Stability Experiments

Dasiglucagon was dissolved in MilliQ water to achieve a stock concentration of 25 mg/mL. Formulations were prepared by aliquoting peptide stock solution (937 µL) into tubes followed by adding the a stock solution of buffer component and then adding MiliQ water to 90 % of the volume. The pH was measured and adjusted if needed before adding MiliQ water to get the final volume. All formulations were then filtered using a Minisart high flow 0.2um filter. The filtered solution was sparged with nitrogen and aliquoted into vials with 1.0 mL in each vial. The vials were crimped and placed in stability chambers.

The formulations were tested under accelerated conditions at 5°C and 25°C and tested at 0, 9, 13, 26, 34, 39 and 52 weeks. The formulations had their purity determined by HPLC and the profile of the degradation products was determined by LC-MS analysis.

The composition of the single-dose (SD) formulations of the present invention are presented in **Table 1** and **Table 5.** All formulations contain mg/mL dasiglucagon and were formulated at pH 6.5. Formulations 1 to 3 investigated the effect of varying the amount of sodium chloride in the formulations, while Formulations 4 to 10 investigated the effect of the buffer used to make the formulations. Formulation 11 is a control formulation in Milli-Q water and Formulation 12 is designed as a multi-dose formulation included in this study by way of comparison with the single dose formulations. The composition of Formulation 12 (MD formulation) is pH 6.5, 4.0 mg/mL of the glucagon analogue, 50 mM TRIS, 90 mM NaCl and 3.15 mg/mL meta-cresol.

The composition of the multi-dose formulations of the present invention are presented in **Tables 2 to 4** and **6.** The formulations all includes meta-cresol and 4 mg/mL dasiglucagon and were formulated at pH 6.0 or pH 6.5. **Tables 2a, 3a** and **4a** report the results of HPLC assays. **Tables 2b, 3b,** and **4b** report result of SEC assays.

The formulations in **Table 2** investigated the effect of the use of different buffers (Tris, Bis-TRIS, MES, sodium citrate, sodium acetate and sodium succinate) on the stability of the formulations at pH 6.0 and pH 6.5 and two concentrations of sodium chloride (90mM and 125mM) as an isotonicity modifier.

The formulations in **Table 3. Table 4** and **Table 6** are similar to those set out in Table 2 and investigate the effect of using combinations of buffers on the stability of the formulations at pH 6.0 and pH 6.5 and at one concentrations of sodium chloride (75mM) as an isotonicity modifier.

### HPLC

The purity of the dasiglucagon samples was determined by HPLC. The samples were analyzed on Dionex Ultimate3000 HPLC system. Chromeleon was used as the analyst software. Dasiglucagon was separated from the degradation products using a Kinetex C8 column (150 mm x 4.6 mm, 2.6 µm). The mobile phase consisted of A: 0.45% TFA (V/V) in MilliQ Water (MQW) and B: 0.45% TFA (V/V) in MeCN/MQW (90:10). The gradient was 35 to 38 %B over 40 minutes with a flow of 0.5 mL/min. The column oven temperature was set to 30°C, the detector wave length was set to 220 nm and 2 µL was injected.

### SEC

Size Exclusion Chromatography (SEC) experiments were carried out on a Dionex Ultimate 3000 HPLC system (Thermo Fisher), using isocratic elution with a flow rate of 0.5 mL/min to assay the formulations for the presence of oligomers. The mobile phase consisted of 0.1% TFA, 45% acetonitrile in MQW. A wavelength of 215 nm was used for detection. Injection amount was 2 µg of peptide. The column used for SEC analysis was a TSKgel SuperSW2000 (TOSOH Corporation), 4µm, 30 x 4.6 mm and the column temperature was 25°C. Runtime was 12 minutes.

### Results

After 52 weeks storage under accelerated conditions at 25°C the following results were obtained for the % purity of the dasiglucagon remaining in the formulations.

The stability studies showed that TRIS, Bis-TRIS, ACES and MES buffers all produced acceptable levels of stability after storage for 52 weeks at 25°C with 80% or more of the dasiglucagon remaining intact despite the accelerated stress conditions used for the experiments, relative to other buffers and controls (Milli-Q water), taking account of the accelerated storage conditions used in the experiments.

For the multi-dose formulations including meta-cresol, TRIS, Bis-TRIS, ACES or MES, and well as citrate, acetate or succinate buffer provided formulations with good levels of stability as assessed by HPLC and SEC.

**Table 5**

| | | **Time, weeks at 25°C, accelerated storage, % dasiglucagon remaining** | | |
|---|---|---|---|---|
| **Formulation No:** | **Composition** | **0** | **13** | **52** |
| 1 | TRIS, 175mM NaCl | 100 | 96 | 83 |
| 2 | TRIS, 500mM NaCl | 100 | 95 | 84 |
| 3 | TRIS, 90mM NaCl | 100 | 96 | 83 |
| 4 | TRIS, 0mM NaCl | 100 | 95 | 84 |
| 5 | Phosphate | 100 | 93 | 76 |
| 6 | Histidine | 100 | 90 | 69 |
| 7 | Bis-TRIS | 100 | 95 | 81 |
| 8 | ACES | 100 | 96 | 83 |
| 9 | Maleic acid | 100 | 91 | 57 |
| 10 | MES | 100 | 90 | 85 |
| 11 | MQW, pH 6.5 | 100 | 74 | n.d. |
| 12 | Multi-Dose lead | 100 | n.d. | 83 |

**Table 5b**

| | | **Time, weeks at 25°C, accelerated storage, % oligomers** | | |
|---|---|---|---|---|
| **Formulation No:** | **Composition** | **0** | **13** | **52** |
| 1 | TRIS, 175mM NaCl | 0.13 | 0.31 | 0.61 |
| 2 | TRIS, 500mM NaCl | 0.13 | 0.36 | 0.74 |
| 3 | TRIS, 90mM NaCl | 0.17 | 0.3 | 0.57 |
| 4 | TRIS, 0mM NaCl | 0.14 | 0.24 | 0.51 |
| 5 | Phosphate | 0.15 | 0.59 | 0.73 |
| 6 | Histidine | 0.12 | 0.42 | 0.84 |
| 7 | Bis-TRIS | 0.12 | 0.53 | 2.81 |
| 8 | ACES | 0.16 | 0.19 | 0.46 |
| 9 | Maleic acid | 0.16 | 0.55 | 4.75 |
| 10 | MES | 0.14 | 0.43 | 0.74 |
| 11 | MQW, pH 6.5 | 0.05 | 0.26 | 0.26 |
| 12 | Multi-Dose lead | 0.19 | n.d. | 1.4 |

The observations are surprising given the challenges reported in the prior art regarding the stabilisation of glucagon formulations including water.

The results are also unexpected as while TRIS and Bis-TRIS have similar chemical structures, the structures of ACES and MES are different.

The experiments also showed that commonly used buffers in liquid peptide formulations, namely phosphate, histidine and maleic acid failed to provide formulations that were capable of long term storage (75%, 68% and 57%) of the dasiglucagon remaining intact, respectively.

The experiments further demonstrate that it is possible to include suitable amounts of NaCl as a tonicity modifier without affecting the stability provided by with the four buffers.

The data show a similar trend of loss of dasiglucagon between the single-dose (SD) formulations (0.31% per week, formulation 1) and the multi-dose (MS) formulation (0.32% per week, formulation 12).

The experiments also demonstrate that it is possible to include a preservative (meta-cresol) in the formulations while preserving storage stability, allowing the development of multi-dose formulations and formulations for use in connection with delivery devices.

### Example 2 - Dasiglucagon ameliorates postprandial hypoglycaemia after Roux-en-Y gastric bypass

Postprandial hypoglycaemia is a frequent and debilitating complication following Roux-en-Y gastric bypass (RYGB), and no effective treatment exists. In a proof-of-concept study, we investigated the effects of dasiglucagon, a novel, stable glucagon analogue, on postprandial hypoglycaemia after RYGB. The primary aim of the present study was to examine the effects of two doses of dasiglucagon on the postprandial nadir plasma glucose concentration (PG) and time spent in hypoglycaemia (<3.9 mmol/l) in RYGB-operated individuals with confirmed postprandial hypoglycaemia.

### Material and Methods

Ten RYGB-operated individuals (two males, eight females; BMI 34.6 (21.8-39.0) kg/m²; age 46 (29-67) years; HbA_{1c} 32 (29-42) mmol/mol; time since operation 9.3 (6-11.5) years; weight loss since operation 53.9 (25.1-92.0) kg)) with confirmed symptomatic postprandial hypoglycaemia (PG <3.5 mmol/I verified by a 6-day continuous glucose monitoring) completed a double-blinded, randomised, crossover study comprising three separate treatment days, each including a standardised liquid mixed meal test (25 kJ per kg body mass; 50 E% carbohydrates, 35 E% fat and 15 E% protein). A subcutaneous injection of either placebo, 80 or 200 µg dasiglucagon (D_{80µg} and D_{200µg}) was administered after the postprandial PG peak, approximately 10 minutes before the projected time point where the PG returned to fasting levels using a subject-specific linear regression model. Blood sampling and assessment of hypoglycaemic symptoms, by the Edinburgh Hypoglycaemia Symptom Scale, were performed at fixed time intervals. Data were analysed using linear mixed models and Tukey's corrections model for multiple comparisons.

Results

Compared with placebo, treatment with both D_{80µg} and D_{200µg} significantly increased nadir PG (placebo: 3.0±0.2 mmol/l; D_{80µg}: 3.9±0.3 mmol/l; D_{200µg}: 4.5±0.2 mmol/l; *p*=0.002 and p=0.0002) and PG incremental AUC (iAUC₇₀₋₂₄₀ₘᵢₙ) after drug administration (placebo: 752±19 min×mmol/l; D_{80µg}: 917±22 min×mmol/l; D_{200µg}: 992±28 min×mmol/l; *p*<0.0001 and p<0.0001). Moreover, both doses reduced time spent in hypoglycaemia compared to placebo (<3.9 mmol/l) (placebo: 62.0±8 min; D_{80µg}: 27.5±12 min; D_{200µg}: 14.0±9 min; p=0.05 and p=0.003). Five participants experienced level 2 hypoglycaemia (<3.1 mmol/l) after placebo administration, whereas none of the participants experienced this after administration of D_{200µg} (time spent in level 2 hypoglycaemia (n=5): placebo: 31.0±5 min; D_{80µg}: 7±5 min; D_{200µg}: 0.0±0 min; p>0.05 and p=0.01). There were no significant changes in hypoglycaemic symptoms between the three study days.

### Conclusion

A single dose administration of dasiglucagon effectively ameliorates postprandial hypoglycaemia and represents a promising new therapeutic option for management of postprandial hypoglycaemia after RYGB.

### References

WO 2014/016300 (Zealand Pharma A/S)
Hövelmann et al, Diabetes Care, 2017 (https://doi.org/10.2337/dc17-1402).
WO 2017/053822 (Xeris Pharmaceuticals, Inc.)
WO 2014/124151 (Xeris Pharmaceuticals, Inc.)
WO 2016/133863 (Eli Lilly and Company)
WO 2019/110838 (Adocia)
WO 2008/084237 (Arecor Limited)

**Table 1**

| Formulation **#** | **Peptide Conc.** | **pH** | **Excipient A** | **Excipient B** | **Excipient C** | **Excipient D** | **Excipient E** | **Excipient F** | **Excipient G** | **Excipient H** |
|---|---|---|---|---|---|---|---|---|---|---|
| | **[mg/mL]** | | **TRIS** | **Phosphate** | **Histidine** | **BIS-TRIS** | **ACES** | **Maleic Acid** | **MES** | **NaCl** |
| | | | **[mM]** | **[mM]** | **[mM]** | **[mM]** | **[mM]** | **[mM]** | **[mM]** | **[mM]** |
| **1** | 1 | 6.5 | 50 | | | | | | | 175 |
| **2** | 1 | 6.5 | 50 | | | | | | | 500 |
| **3** | 1 | 6.5 | 50 | | | | | | | 90 |
| **4** | 1 | 6.5 | 50 | | | | | | | |
| **5** | 1 | 6.5 | | 50 | | | | | | |
| **6** | 1 | 6.5 | | | 50 | | | | | |
| **7** | 1 | 6.5 | | | | 50 | | | | |
| **8** | 1 | 6.5 | | | | | 50 | | | |
| **9** | 1 | 6.5 | | | | | | 50 | | |
| **10** | 1 | 6.5 | | | | | | | 50 | |
| **11** | 1 | 6.5 | | | | | | | | |

Formulation 12 (MD formulation) is pH 6.5, 4.0 mg/mL dasiglucagon, 50 mM TRIS, 90 mM NaCl and 3.15 mg/mL meta-cresol.

**Table 2**

| **Formulation #** | **Peptide Conc.** | **pH** | **Excipient A** | **Excipient B** | **Excipient C** | **Excipient D** | **Excipient E** | **Excipient F** | **Excipient G** | **Excipient H** |
|---|---|---|---|---|---|---|---|---|---|---|
| | **[mg/mL]** | | **Tris** | **Bis-Tris** | **MES** | **Na Citrate** | **Na Acetate** | **Na Succinate** | **m-cresol** | **NaCl** |
| | | | **[mM]** | **[mM]** | **[mM]** | **[mM]** | **[mM]** | **[mM]** | **[mg/mL]** | **[mM]** |
| **1** | 4 | 6.5 | 50 | | | | | | 3.15 | 90 |
| **2** | 4 | 6.0 | 50 | | | | | | 3.15 | 90 |
| **3** | 4 | 6.5 | | 50 | | | | | 3.15 | 90 |
| **4** | 4 | 6.0 | | 50 | | | | | 3.15 | 90 |
| **5** | 4 | 6.5 | | | 50 | | | | 3.15 | 90 |
| **6** | 4 | 6.0 | | | 50 | | | | 3.15 | 90 |
| **7** | 4 | 6.5 | | | | 15 | | | 3.15 | 125 |
| **8** | 4 | 6.0 | | | | 15 | | | 3.15 | 125 |
| **9** | 4 | 6.5 | | | | | 15 | | 3.15 | 125 |
| **10** | 4 | 6.0 | | | | | 15 | | 3.15 | 125 |
| **11** | 4 | 6.5 | | | | | | 15 | 3.15 | 125 |
| **12** | 4 | 6.0 | | | | | | 15 | 3.15 | 125 |

**Table 2a**

| | **HPLC Assay** | **Time, weeks at 5°C, % dasiglucagon remaining** | | |
|---|---|---|---|---|
| **Formulation No:** | **Composition** | **0** | **26** | **52** |
| 1 | Tris 50 mM, NaCl 90 mM, m-cresol 3.15 mg/mL, pH 6.5 (reference formulation) | 100 | 99.75 | 98.29 |
| 2 | Tris 50 mM, NaCl 90 mM, m-cresol 3.15 mg/mL, pH 6.0 (reference formulation pH 6.0) | 100 | 99.54 | 98.54 |
| 3 | Bis-Tris 50 mM, NaCl 90 mM, m-cresol 3.15 mg/mL, pH 6.5 | 100 | 99.30 | 97.64 |
| 4 | Bis-Tris 50 mM, NaCl 90 mM, m-cresol 3.15 mg/mL, pH 6.0 | 100 | * | * |
| 5 | MES 50 mM, NaCl 90 mM, m-cresol 3.15 mg/mL, pH 6.5 | 100 | 99.43 | 98.40 |
| 6 | MES 50 mM, NaCl 90 mM, m-cresol 3.15 mg/mL, pH 6.0 | 100 | * | * |
| 7 | Na Citrate 15 mM, NaCl 90 mM, m-cresol 3.15 mg/mL, pH 6.5 | 100 | 99.64 | 98.07 |
| 8 | Na Citrate 15 mM, NaCl 90 mM, m-cresol 3.15 mg/mL, pH 6.0 | 100 | * | * |
| 9 | Na Acetate 15 mM, NaCl 90 mM, m-cresol 3.15 mg/mL, pH 6.5 | 100 | 99.11 | 97.15 |
| 10 | Na Acetate 15 mM, NaCl 90 mM, m-cresol 3.15 mg/mL, pH 6.0 | 100 | * | * |
| 11 | Na Succinate 15 mM, NaCl 90 mM, m-cresol 3.15 mg/mL, pH 6.5 | 100 | 99.56 | 98.61 |
| 12 | Na Succinate 15 mM, NaCl 90 mM, m-cresol 3.15 mg/mL, pH 6.0 | 100 | * | * |

| | | | | |
|---|---|---|---|---|
| *Precipitation at 5°C | | | | |

**Table 2b**

| | **SEC Assay** | **Time, weeks at 5°C, % oligomers** | | |
|---|---|---|---|---|
| **Formulation No:** | **Composition** | **0** | **26** | **52** |
| 1 | Tris 50 mM, NaCl 90 mM, m-cresol 3.15 mg/mL, pH 6.5 | 0.16 | 0.25 | 0.26 |
| 2 | Tris 50 mM, NaCl 90 mM, m-cresol 3.15 mg/mL, pH 6.0 | 0.16 | 0.25 | 0.27 |
| 3 | Bis-Tris 50 mM, NaCl 90 mM, m-cresol 3.15 mg/mL, pH 6.5 | 0.16 | 0.37 | 0.52 |
| 4 | Bis-Tris 50 mM, NaCl 90 mM, m-cresol 3.15 mg/mL, pH 6.0 | 0.15 | * | * |
| 5 | MES 50 mM, NaCl 90 mM, m-cresol 3.15 mg/mL, pH 6.5 | 0.15 | 0.27 | 0.26 |
| 6 | MES 50 mM, NaCl 90 mM, m-cresol 3.15 mg/mL, pH 6.0 | 0.15 | * | * |
| 7 | Na Citrate 15 mM, NaCl 90 mM, m-cresol 3.15 mg/mL, pH 6.5 | 0.15 | 0.19 | 0.17 |
| 8 | Na Citrate 15 mM, NaCl 90 mM, m-cresol 3.15 mg/mL, pH 6.0 | 0.14 | * | * |
| 9 | Na Acetate 15 mM, NaCl 90 mM, m-cresol 3.15 mg/mL, pH 6.5 | 0.15 | 0.24 | 0.24 |
| 10 | Na Acetate 15 mM, NaCl 90 mM, m-cresol 3.15 mg/mL, pH 6.0 | 0.15 | * | * |
| 11 | Na Succinate 15 mM, NaCl 90 mM, m-cresol 3.15 mg/mL, pH 6.5 | 0.15 | 0.28 | 0.33 |
| 12 | Na Succinate 15 mM, NaCl 90 mM, m-cresol 3.15 mg/mL, pH 6.0 | 0.15 | * | * |

| | | | | |
|---|---|---|---|---|
| *Precipitation at 5°C | | | | |

**Table 2c**

| | **HPLC Assay** | **Time, weeks at 25°C, accelerated storage, % dasiglucagon remaining** | | |
|---|---|---|---|---|
| **Formulation No:** | **Composition** | **0** | **13** | **26** |
| 1 | Tris 50 mM, NaCl 90 mM, m-cresol 3.15 mg/mL, pH 6.5 (control formulation) | 100 | 95.39 | 91.35 |
| 2 | Tris 50 mM, NaCl 90 mM, m-cresol 3.15 mg/mL, pH 6.0 (control formulation pH 6.0) | 100 | 96.11 | 92.17 |
| 3 | Bis-Tris 50 mM, NaCl 90 mM, m-cresol 3.15 mg/mL, pH 6.5 | 100 | 94.54 | 89.19 |
| 4 | Bis-Tris 50 mM, NaCl 90 mM, m-cresol 3.15 mg/mL, pH 6.0 | 100 | 95.46 | 91.47 |
| 5 | MES 50 mM, NaCl 90 mM, m-cresol 3.15 mg/mL, pH 6.5 | 100 | 95.98 | 92.10 |
| 6 | MES 50 mM, NaCl 90 mM, m-cresol 3.15 mg/mL, pH 6.0 | 100 | 96.11 | 92.46 |
| 7 | Na Citrate 15 mM, NaCl 90 mM, m-cresol 3.15 mg/mL, pH 6.5 | 100 | 95.42 | 91.89 |
| 8 | Na Citrate 15 mM, NaCl 90 mM, m-cresol 3.15 mg/mL, pH 6.0 | 100 | 95.66 | 91.56 |
| 9 | Na Acetate 15 mM, NaCl 90 mM, m-cresol 3.15 mg/mL, pH 6.5 | 100 | 94.89 | 90.15 |
| 10 | Na Acetate 15 mM, NaCl 90 mM, m-cresol 3.15 mg/mL, pH 6.0 | 100 | 95.91 | 91.81 |
| 11 | Na Succinate 15 mM, NaCl 90 mM, m-cresol 3.15 mg/mL, pH 6.5 | 100 | 95.51 | 91.42 |
| 12 | Na Succinate 15 mM, NaCl 90 mM, m-cresol 3.15 mg/mL, pH 6.0 | 100 | 96.22 | 92.18 |

**Table 2d**

| | **SEC Assay** | **Time, weeks at 25°C, accelerated storage, % oligomers** | | |
|---|---|---|---|---|
| **Formulation No:** | **Composition** | **0** | **13** | **26** |
| 1 | Tris 50 mM, NaCl 90 mM, m-cresol 3.15 mg/mL, pH 6.5 | 0.16 | 0.40 | 0.62 |
| 2 | Tris 50 mM, NaCl 90 mM, m-cresol 3.15 mg/mL, pH 6.0 | 0.16 | 0.41 | 0.59 |
| 3 | Bis-Tris 50 mM, NaCl 90 mM, m-cresol 3.15 mg/mL, pH 6.5 | 0.16 | 1.16 | 2.98 |
| 4 | Bis-Tris 50 mM, NaCl 90 mM, m-cresol 3.15 mg/mL, pH 6.0 | 0.15 | 0.77 | 1.52 |
| 5 | MES 50 mM, NaCl 90 mM, m-cresol 3.15 mg/mL, pH 6.5 | 0.15 | 0.42 | 0.64 |
| 6 | MES 50 mM, NaCl 90 mM, m-cresol 3.15 mg/mL, pH 6.0 | 0.15 | 0.45 | 0.68 |
| 7 | Na Citrate 15 mM, NaCl 90 mM, m-cresol 3.15 mg/mL, pH 6.5 | 0.15 | 0.26 | 0.35 |
| 8 | Na Citrate 15 mM, NaCl 90 mM, m-cresol 3.15 mg/mL, pH 6.0 | 0.14 | 0.26 | 0.35 |
| 9 | Na Acetate 15 mM, NaCl 90 mM, m-cresol 3.15 mg/mL, pH 6.5 | 0.15 | 0.38 | 0.64 |
| 10 | Na Acetate 15 mM, NaCl 90 mM, m-cresol 3.15 mg/mL, pH 6.0 | 0.15 | 0.50 | 0.76 |
| 11 | Na Succinate 15 mM, NaCl 90 mM, m-cresol 3.15 mg/mL, pH 6.5 | 0.15 | 0.52 | 0.86 |
| 12 | Na Succinate 15 mM, NaCl 90 mM, m-cresol 3.15 mg/mL, pH 6.0 | 0.15 | 0.56 | 1.02 |

**Table 3**

| **Formulation #** | **Peptide Conc.** | **pH** | **Excipient A** | **Excipient B** | **Excipient D** | **Excipient E** | **Excipient F** | **Excipient G** | **Excipient H** |
|---|---|---|---|---|---|---|---|---|---|
| | **[mg/mL]** | | **Tris** | **Bis-Tris** | **Na Citrate** | **Na Acetate** | **Na Succinate** | **m-cresol** | **NaCl** |
| | | | **[mM]** | **[mM]** | **[mM]** | **[mM]** | **[mM]** | **[mg/mL]** | **[mM]** |
| **1** | 4 | 6.5 | 50 | | 15 | | | 3.15 | 75 |
| **2** | 4 | 6.0 | 50 | | 15 | | | 3.15 | 75 |
| **3** | 4 | 6.5 | 50 | | | 15 | | 3.15 | 75 |
| **4** | 4 | 6.0 | 50 | | | 15 | | 3.15 | 75 |
| **5** | 4 | 6.5 | 50 | | | | 15 | 3.15 | 75 |
| **6** | 4 | 6.0 | 50 | | | | 15 | 3.15 | 75 |
| **7** | 4 | 6.5 | | 50 | 15 | | | 3.15 | 75 |
| **8** | 4 | 6.0 | | 50 | 15 | | | 3.15 | 75 |
| **9** | 4 | 6.5 | | 50 | | 15 | | 3.15 | 75 |
| **10** | 4 | 6.0 | | 50 | | 15 | | 3.15 | 75 |
| **11** | 4 | 6.5 | | 50 | | | 15 | 3.15 | 75 |
| **12** | 4 | 6.0 | | 50 | | | 15 | 3.15 | 75 |

**Table 3a**

| | **HPLC Assay** | **Time, weeks at 5°C, % dasiglucagon remaining** | | |
|---|---|---|---|---|
| **Formulation No:** | **Composition** | **0** | **26** | **52** |
| 1 | Tris 50 mM, Na citrate 15 mM, NaCl 90 mM, m-cresol 3.15 mg/mL, pH 6.5 | 100 | 99.17 | 98.43 |
| 2 | Tris 50 mM, Na citrate 15 mM, NaCl 90 mM, m-cresol 3.15 mg/mL, pH 6.0 | 100 | * | * |
| 3 | Tris 50 mM, Na acetate 15 mM, NaCl 90 mM, m-cresol 3.15 mg/mL, pH 6.5 | 100 | 99.06 | 98.38 |
| 4 | Tris 50 mM, Na acetate 15 mM, NaCl 90 mM, m-cresol 3.15 mg/mL, pH 6.0 | 100 | * | * |
| 5 | Tris 50 mM, Na succinate 15 mM, NaCl 90 mM, m-cresol 3.15 mg/mL, pH 6.5 | 100 | 99.17 | 98.13 |
| 6 | Tris 50 mM, Na succinate 15 mM, NaCl 90 mM, m-cresol 3.15 mg/mL, pH 6.0 | 100 | * | * |
| 7 | Bis-Tris 50 mM, Na citrate 15 mM, NaCl 90 mM, m-cresol 3.15 mg/mL, pH 6.5 | 100 | 99.06 | 98.09 |
| 8 | Bis-Tris 50 mM, Na citrate 15 mM, NaCl 90 mM, m-cresol 3.15 mg/mL, pH 6.0 | 100 | * | * |
| 9 | Bis-Tris 50 mM, Na acetate 15 mM, NaCl 90 mM, m-cresol 3.15 mg/mL, pH 6.5 | 100 | 99.15 | 97.55 |
| 10 | Bis-Tris 50 mM, Na acetate 15 mM, NaCl 90 mM, m-cresol 3.15 mg/mL, pH 6.0 | 100 | * | * |
| 11 | Bis-Tris 50 mM, Na succinate 15 mM, NaCl 90 mM, m-cresol 3.15 mg/mL, pH 6.5 | 100 | 99.03 | 98.03 |
| 12 | Bis-Tris 50 mM, Na succinate 15 mM, NaCl 90 mM, m-cresol 3.15 mg/mL, pH 6.0 | 100 | * | * |

| | | | | |
|---|---|---|---|---|
| *Precipitation at 5°C | | | | |

**Table 3b**

| | **SEC Assay** | **Time, weeks at 5°C, % oligomers** | | |
|---|---|---|---|---|
| **Formulation No:** | **Composition** | **0** | **26** | **52** |
| 1 | Tris 50 mM, Na citrate 15 mM, NaCl 90 mM, m-cresol 3.15 mg/mL, pH 6.5 | 0.15 | 0.18 | 0.16 |
| 2 | Tris 50 mM, Na citrate 15 mM, NaCl 90 mM, m-cresol 3.15 mg/mL, pH 6.0 | 0.15 | * | * |
| 3 | Tris 50 mM, Na acetate 15 mM, NaCl 90 mM, m-cresol 3.15 mg/mL, pH 6.5 | 0.15 | 0.25 | 0.27 |
| 4 | Tris 50 mM, Na actate 15 mM, NaCl 90 mM, m-cresol 3.15 mg/mL, pH 6.0 | 0.14 | * | * |
| 5 | Tris 50 mM, Na succinate 15 mM, NaCl 90 mM, m-cresol 3.15 mg/mL, pH 6.5 | 0.15 | 0.25 | 0.25 |
| 6 | Tris 50 mM, Na succinate 15 mM, NaCl 90 mM, m-cresol 3.15 mg/mL, pH 6.0 | 0.14 | * | * |
| 7 | Bis-Tris 50 mM, Na citrate 15 mM, NaCl 90 mM, m-cresol 3.15 mg/mL, pH 6.5 | 0.15 | 0.32 | 0.35 |
| 8 | Bis-Tris 50 mM, Na citrate 15 mM, NaCl 90 mM, m-cresol 3.15 mg/mL, pH 6.0 | 0.14 | * | * |
| 9 | Bis-Tris 50 mM, Na acetate 15 mM, NaCl 90 mM, m-cresol 3.15 mg/mL, pH 6.5 | 0.15 | 0.35 | 0.51 |
| 10 | Bis-Tris 50 mM, Na acetate 15 mM, NaCl 90 mM, m-cresol 3.15 mg/mL, pH 6.0 | 0.15 | * | * |
| 11 | Bis-Tris 50 mM, Na succinate 15 mM, NaCl 90 mM, m-cresol 3.15 mg/mL, pH 6.5 | 0.15 | 0.37 | 0.55 |
| 12 | Bis-Tris 50 mM, Na succinate 15 mM, NaCl 90 mM, m-cresol 3.15 mg/mL, pH 6.0 | 0.16 | * | * |

| | | | | |
|---|---|---|---|---|
| *Precipitation at 5°C | | | | |

**Table 3c**

| | **HPLC Assay** | **Time, weeks at 25°C, accelerated storage, % dasiglucagon remaining** | | |
|---|---|---|---|---|
| **Formulation No:** | **Composition** | **0** | **13** | **26** |
| 1 | Tris 50 mM, Na citrate 15 mM, NaCl 90 mM, m-cresol 3.15 mg/mL, pH 6.5 | 100 | 95.22 | 90.56 |
| 2 | Tris 50 mM, Na citrate 15 mM, NaCl 90 mM, m-cresol 3.15 mg/mL, pH 6.0 | 100 | 95.36 | 90.07 |
| 3 | Tris 50 mM, Na acetate 15 mM, NaCl 90 mM, m-cresol 3.15 mg/mL, pH 6.5 | 100 | 95.55 | 91.03 |
| 4 | Tris 50 mM, Na acetate 15 mM, NaCl 90 mM, m-cresol 3.15 mg/mL, pH 6.0 | 100 | 95.81 | 91.42 |
| 5 | Tris 50 mM, Na succinate 15 mM, NaCl 90 mM, m-cresol 3.15 mg/mL, pH 6.5 | 100 | 95.05 | 91.20 |
| 6 | Tris 50 mM, Na succinate 15 mM, NaCl 90 mM, m-cresol 3.15 mg/mL, pH 6.0 | 100 | 95.59 | 90.96 |
| 7 | Bis-Tris 50 mM, Na citrate 15 mM, NaCl 90 mM, m-cresol 3.15 mg/mL, pH 6.5 | 100 | 95.14 | 90.43 |
| 8 | Bis-Tris 50 mM, Na citrate 15 mM, NaCl 90 mM, m-cresol 3.15 mg/mL, pH 6.0 | 100 | 95.05 | 90.31 |
| 9 | Bis-Tris 50 mM, Na acetate 15 mM, NaCl 90 mM, m-cresol 3.15 mg/mL, pH 6.5 | 100 | 93.86 | n.d. |
| 10 | Bis-Tris 50 mM, Na acetate 15 mM, NaCl 90 mM, m-cresol 3.15 mg/mL, pH 6.0 | 100 | 95.24 | n.d. |
| 11 | Bis-Tris 50 mM, Na succinate 15 mM, NaCl 90 mM, m-cresol 3.15 mg/mL, pH 6.5 | 100 | 93.97 | n.d. |
| 12 | Bis-Tris 50 mM, Na succinate 15 mM, NaCl 90 mM, m-cresol 3.15 mg/mL, pH 6.0 | 100 | 95.00 | n.d. |

**Table 3d**

| | **SEC Assay** | **Time, weeks at 25°C, accelerated storage, % oligomers** | | |
|---|---|---|---|---|
| **Formulation No:** | **Composition** | **0** | **13** | **26** |
| 1 | Tris 50 mM, Na citrate 15 mM, NaCl 90 mM, m-cresol 3.15 mg/mL, pH 6.5 | 0.15 | 0.23 | 0.32 |
| 2 | Tris 50 mM, Na citrate 15 mM, NaCl 90 mM, m-cresol 3.15 mg/mL, pH 6.0 | 0.15 | 0.25 | 0.34 |
| 3 | Tris 50 mM, Na acetate 15 mM, NaCl 90 mM, m-cresol 3.15 mg/mL, pH 6.5 | 0.15 | 0.38 | 0.60 |
| 4 | Tris 50 mM, Na actate 15 mM, NaCl 90 mM, m-cresol 3.15 mg/mL, pH 6.0 | 0.14 | 0.29 | 0.47 |
| 5 | Tris 50 mM, Na succinate 15 mM, NaCl 90 mM, m-cresol 3.15 mg/mL, pH 6.5 | 0.15 | 0.39 | 0.62 |
| 6 | Tris 50 mM, Na succinate 15 mM, NaCl 90 mM, m-cresol 3.15 mg/mL, pH 6.0 | 0.14 | 0.27 | 0.38 |
| 7 | Bis-Tris 50 mM, Na citrate 15 mM, NaCl 90 mM, m-cresol 3.15 mg/mL, pH 6.5 | 0.15 | 0.73 | 1.83 |
| 8 | Bis-Tris 50 mM, Na citrate 15 mM, NaCl 90 mM, m-cresol 3.15 mg/mL, pH 6.0 | 0.14 | 0.57 | 1.10 |
| 9 | Bis-Tris 50 mM, Na acetate 15 mM, NaCl 90 mM, m-cresol 3.15 mg/mL, pH 6.5 | 0.15 | 1.40 | 3.75 |
| 10 | Bis-Tris 50 mM, Na actate 15 mM, NaCl 90 mM, m-cresol 3.15 mg/mL, pH 6.0 | 0.15 | 0.83 | 1.64 |
| 11 | Bis-Tris 50 mM, Na succinate 15 mM, NaCl 90 mM, m-cresol 3.15 mg/mL, pH 6.5 | 0.15 | 1.50 | 4.50 |
| 12 | Bis-Tris 50 mM, Na succinate 15 mM, NaCl 90 mM, m-cresol 3.15 mg/mL, pH 6.0 | 0.16 | 0.89 | 1.85 |

**Table 4**

| **Formulation #** | **Peptide Conc.** | **pH** | **Excipient A** | **Excipient B** | **Excipient C** | **Excipient D** | **Excipient E** | **Excipient F** |
|---|---|---|---|---|---|---|---|---|
| | **[mg/mL]** | | **MES** | **Na Citrate** | **Na Acetate** | **Na Succinate** | **m-cresol** | **NaCl** |
| | | | **[mM]** | **[mM]** | **[mM]** | **[mM]** | **[mM]** | **[mM]** |
| **1** | 4 | 6.5 | 50 | 15 | | | 3.15 | 75 |
| **2** | 4 | 6.0 | 50 | 15 | | | 3.15 | 75 |
| **3** | 4 | 6.5 | 50 | | 15 | | 3.15 | 75 |
| **4** | 4 | 6.0 | 50 | | 15 | | 3.15 | 75 |
| **5** | 4 | 6.5 | 50 | | | 15 | 3.15 | 75 |
| **6** | 4 | 6.0 | 50 | | | 15 | 3.15 | 75 |

**Table 4a**

| | **HPLC Assay** | **Time, weeks at 5°C, % dasiglucagon remaining** | | |
|---|---|---|---|---|
| **Formulation No:** | **Composition** | **0** | **26** | **52** |
| 1 | MES 50 mM, Na citrate 15 mM, NaCl 90 mM, m-cresol 3.15 mg/mL, pH 6.5 | 100 | 99.86 | 99.44 |
| 2 | MES 50 mM, Na citrate 15 mM, NaCl 90 mM, m-cresol 3.15 mg/mL, pH 6.0 | 100 | * | * |
| 3 | MES 50 mM, Na acetate 15 mM, NaCl 90 mM, m-cresol 3.15 mg/mL, pH 6.5 | 100 | 99.65 | 99.65 |
| 4 | MES 50 mM, Na acetate 15 mM, NaCl 90 mM, m-cresol 3.15 mg/mL, pH 6.0 | 100 | * | * |
| 5 | MES 50 mM, Na succinate 15 mM, NaCl 90 mM, m-cresol 3.15 mg/mL, pH 6.5 | 100 | 99.72 | 99.60 |
| 6 | MES 50 mM, Na succinate 15 mM, NaCl 90 mM, m-cresol 3.15 mg/mL, pH 6.0 | 100 | * | * |

| | | | | |
|---|---|---|---|---|
| *Precipitation at 5°C | | | | |

**Table 4b**

| | **SEC Assay** | **Time, weeks at 5°C, % oligomers** | | |
|---|---|---|---|---|
| **Formulation No:** | **Composition** | **0** | **26** | **52** |
| 1 | MES 50 mM, Na citrate 15 mM, NaCl 90 mM, m-cresol 3.15 mg/mL, pH 6.5 | 0.14 | 0.19 | 0.24 |
| 2 | MES 50 mM, Na citrate 15 mM, NaCl 90 mM, m-cresol 3.15 mg/mL, pH 6.0 | 0.14 | * | * |
| 3 | MES 50 mM, Na acetate 15 mM, NaCl 90 mM, m-cresol 3.15 mg/mL, pH 6.5 | 0.14 | 0.22 | 0.25 |
| 4 | MES 50 mM, Na acetate 15 mM, NaCl 90 mM, m-cresol 3.15 mg/mL, pH 6.0 | 0.08 | * | * |
| 5 | MES 50 mM, Na succinate 15 mM, NaCl 90 mM, m-cresol 3.15 mg/mL, pH 6.5 | 0.14 | 0.23 | 0.26 |
| 6 | MES 50 mM, Na succinate 15 mM, NaCl 90 mM, m-cresol 3.15 mg/mL, pH 6.0 | 0.13 | * | * |

| | | | | |
|---|---|---|---|---|
| *Precipitation at 5°C DLS and absorbance were acceptable for all the acetate containing formulations compared to control formulations. | | | | |

**Table 4c**

| | **HPLC Assay** | **Time, weeks at 25°C, accelerated storage, % dasiglucagon remaining** | | |
|---|---|---|---|---|
| **Formulation No:** | **Composition** | **0** | **13** | **26** |
| 1 | MES 50 mM, Na citrate 15 mM, NaCl 90 mM, m-cresol 3.15 mg/mL, pH 6.5 | 100 | 95.90 | 92.18 |
| 2 | MES 50 mM, Na citrate 15 mM, NaCl 90 mM, m-cresol 3.15 mg/mL, pH 6.0 | 100 | 95.62 | 91.20 |
| 3 | MES 50 mM, Na acetate 15 mM, NaCl 90 mM, m-cresol 3.15 mg/mL, pH 6.5 | 100 | 95.92 | 92.36 |
| 4 | MES 50 mM, Na actate 15 mM, NaCl 90 mM, m-cresol 3.15 mg/mL, pH 6.0 | 100 | 95.78 | 92.55 |
| 5 | MES 50 mM, Na succinate 15 mM, NaCl 90 mM, m-cresol 3.15 mg/mL, pH 6.5 | 100 | 96.10 | 92.39 |
| 6 | MES 50 mM, Na succinate 15 mM, NaCl 90 mM, m-cresol 3.15 mg/mL, pH 6.0 | 100 | 95.85 | 92.22 |

**Table 4d**

| | **SEC Assay** | **Time, weeks at 25°C, accelerated storage, % oligomers** | | |
|---|---|---|---|---|
| **Formulation No:** | **Composition** | **0** | **13** | **26** |
| 1 | MES 50 mM, Na citrate 15 mM, NaCl 90 mM, m-cresol 3.15 mg/mL, pH 6.5 | 0.14 | 0.28 | 0.36 |
| 2 | MES 50 mM, Na citrate 15 mM, NaCl 90 mM, m-cresol 3.15 mg/mL, pH 6.0 | 0.14 | 0.29 | 0.38 |
| 3 | MES 50 mM, Na acetate 15 mM, NaCl 90 mM, m-cresol 3.15 mg/mL, pH 6.5 | 0.14 | 0.38 | 0.59 |
| 4 | MES 50 mM, Na actate 15 mM, NaCl 90 mM, m-cresol 3.15 mg/mL, pH 6.0 | 0.08 | 0.38 | 0.62 |
| 5 | MES 50 mM, Na succinate 15 mM, NaCl 90 mM, m-cresol 3.15 mg/mL, pH 6.5 | 0.14 | 0.40 | 0.60 |
| 6 | MES 50 mM, Na succinate 15 mM, NaCl 90 mM, m-cresol 3.15 mg/mL, pH 6.0 | 0.13 | 0.44 | 0.68 |

**Table 6**

| **Formulation #** | **Peptide Conc.** | **pH** | **Excipient A** | **Excipient B** | **Excipient C** | **Excipient D** | **Excipient E** |
|---|---|---|---|---|---|---|---|
| | | | **Tris** | **Phosphate** | **Histidine** | **NaCl** | **m-cresol** |
| | **[mg/mL]** | | **[mM]** | **[mM]** | **[mM]** | **[mM]** | **[mg/mL]** |
| **1** | 4 | 6.5 | 50 | | | 90 | 3.15 |
| **2** | 4 | 6.5 | | 50 | | 90 | 3.15 |
| **3** | 4 | 6.5 | | | 25 | 90 | 3.15 |
| **4** | 4 | 6.5 | 50 | 50 | | 90 | 3.15 |
| **5** | 4 | 6.5 | 50 | | 25 | 90 | 3.15 |
| **6** | 4 | 6.5 | | 50 | 25 | 90 | 3.15 |

**Table 6a**

| | **HPLC Assay** | **Time, days at 40°C, accelerated storage, % dasiglucagon remaining** | | | |
|---|---|---|---|---|---|
| **Formulation No:** | **Composition** | **0** | **7** | **14** | **18** |
| 1 | Tris 50 mM, NaCl 90 mM, m-cresol 3.15 mg/mL, pH 6.5 (reference formulation) | 100 | 97.4 | 95.8 | 95.0 |
| 2 | Phosphate 50 mM, NaCl 90 mM, m-cresol 3.15 mg/mL, pH 6.5 | 100 | 96.6 | 94.4 | 92.9 |
| 3 | Histidine 25 mM, NaCl 90 mM, m-cresol 3.15 mg/mL, pH 6.5 | 100 | 97.5 | 94.1 | 92.7 |
| 4 | Tris 50 mM, Phosphate 50 mM, NaCl 90 mM, m-cresol 3.15 mg/mL, pH 6.5 | 100 | 97.5 | 94.5 | 92.6 |
| 5 | Tris 50 mM, Histidine 25 mM, NaCl 90 mM, m-cresol 3.15 mg/mL, pH 6.5 | 100 | 97.5 | 94.1 | 92.7 |
| 6 | Phosphate 50 mM, Histidine 25 mM, NaCl 90 mM, m-cresol 3.15 mg/mL, pH 6.5 | 100 | 97.6 | 94.2 | 92.2 |

**Table 6b**

| | **SEC Assay** | **Time, days at 40°C, accelerated storage, % oligomers** | | | |
|---|---|---|---|---|---|
| **Formulation No:** | **Composition** | **0** | **7** | **14** | **18** |
| 1 | Tris 50 mM, NaCl 90 mM, m-cresol 3.15 mg/mL, pH 6.5 (reference formulation) | 0.11 | 0.30 | 0.38 | 0.43 |
| 2 | Phosphate 50 mM, NaCl 90 mM, m-cresol 3.15 mg/mL, pH 6.5 | 0.12 | 0.24 | 0.30 | 0.31 |
| 3 | Histidine 25 mM, NaCl 90 mM, m-cresol 3.15 mg/mL, pH 6.5 | 0.1 | 0.44 | 0.85 | 0.93 |
| 4 | Tris 50 mM, Phosphate 50 mM, NaCl 90 mM, m-cresol 3.15 mg/mL, pH 6.5 | 0.12 | 0.21 | 0.27 | 0.29 |
| 5 | Tris 50 mM, Histidine 25 mM, NaCl 90 mM, m-cresol 3.15 mg/mL, pH 6.5 | 0.11 | 0.35 | 0.75 | 0.85 |
| 6 | Phosphate 50 mM, Histidine 25 mM, NaCl 90 mM, m-cresol 3.15 mg/mL, pH 6.5 | 0.1 | 0.18 | 0.28 | 0.29 |

## Claims

1. A stable aqueous liquid pharmaceutical formulation comprising a glucagon analogue, which is:
Hy-HSQGTFTSDYSKYLD-Aib-ARAEEFVKWLEST-OH
or a pharmaceutically acceptable salt thereof;
wherein the formulation comprises:
(a) the glucagon analogue present at a concentration of about 0.5 mg/mL to about 10 mg/mL;
(b) TRIS, ACES or MES is present as a buffer at a concentration of about 25 mM to about 75 mM, and/or citrate, acetate or succinate is present as a buffer at a concentration of about 1 mM to about 30 mM;
(c) sodium chloride present as a tonicity modifier and at a concentration of about 50 mM to about 600 mM; and
(d) a pH of about 5.6 to about 7.0.

2. The stable aqueous liquid formulation of claim 1, wherein the TRIS, ACES or MES buffer directly chemically stabilise the glucagon analogue independent of the pH of the formulation provided by the buffer.

3. The stable aqueous liquid formulation of claim 1 or claim 2, wherein the formulation:
i) provides improved chemical stabilisation relative to a formulation in which the TRIS, ACES, or MES buffer is replaced by a phosphate buffer and/or a histidine buffer of the same concentration and pH evaluated under the same test conditions;
ii) has a degradation profile following 52 weeks storage at 25°C after which the formulation contains one or more of less than 5% of Pyro-Glu 4-29, less than 7% of Trp/Tyr oxidation, less than 4% of Kynurenine, less than 5% of F-4-29 + F5-29 and/or less than 2% of F3.29 after storage for 52 weeks at 25°C, wherein all percentages are determined by HPLC;
iii) has a degradation profile in which the glucagon analogue is free of succinic acid addition to maleic acid;
iv) substantially does not include aprotic polar solvent; and/or
v) substantially does not include dimethyl sulfoxide (DMSO).

4. The stable aqueous liquid formulation of any one of the preceding claims, wherein the water is the sole solvent used to make the aqueous liquid formulation.

5. The stable aqueous liquid formulation of any one of the preceding claims, wherein:
i) the glucagon analogue, or the pharmaceutically acceptable salt thereof, is present at a concentration of about 0.5 mg/mL, 0.6 mg/mL, 0.7 mg/mL, 1.0 mg/mL or about 4.0 mg/mL;
ii) the TRIS, ACES or MES buffer is present as a buffer at a concentration of about 50 mM;
iii) the sodium chloride is present as a tonicity modifier at a concentration of about 150 mM to about 200 mM or at a concentration of about 50 mM to about 150 mM; and/or
iv) the formulation has a pH of about 5.8 to 6.7, optionally wherein the formulation has a pH of about 6.5.

6. The stable aqueous liquid formulation of any one of the preceding claims, wherein the formulation:
i) does not include an ionization stabilizing excipient selected from hydrochloric acid, nitric acid, sulphuric acid or a combination thereof;
ii) is a ready-to-use formulation;
iii) is stable at 2-8°C for at least 6 months, at least 12 months, at least 18 months or at least 24 months, optionally wherein the glucagon analogue in the formulation retains at least about 90% of its biological activity after 18 months of storage 2-8°C;
iv) is sterile and/or free from a reducing agent;
v) is formulated for administration to a subject by injection, optionally wherein the injection is subcutaneous injection.

7. The stable aqueous liquid formulation of any one of the preceding claims, wherein the buffer is TRIS.

8. The stable aqueous liquid formulation of any one of the preceding claims, further comprising a preservative, optionally wherein the preservative is meta-cresol, optionally at a concentration of about 1.0 mg/mL to about 5.0 mg/mL.

9. The stable aqueous liquid formulation of any one of claims 1 to 6, wherein the glucagon analogue, or the pharmaceutically acceptable salt thereof, is present at a concentration of about 0.75 mg/ml to about 1.25 mg/mL, the TRIS, ACES or MES buffer is present at a concentration of about 40 mM to about 60 mM, sodium chloride is present as a tonicity modifier at a concentration of about 150 mM to about 200 mM and the formulation has a pH of about 6.0 to about 6.8,optionally wherein the glucagon analogue, or the pharmaceutically acceptable salt thereof, is present at a concentration of about 1.0 mg/mL, TRIS is present at a concentration of about 50 mM, sodium chloride is present as a tonicity modifier at a concentration of about 175 mM and the formulation has a pH of about 6.5.

10. The stable aqueous liquid formulation of any one claims 1 to 8, wherein:
a) the glucagon analogue, or the pharmaceutically acceptable salt thereof, is present at a concentration of about 3.0 to 5.0 mg/mL, TRIS, ACES or MES buffer is present at a concentration of about 40 mM to about 60 mM, sodium chloride is present as a tonicity modifier at a concentration of about 50 mM to about 150 mM, meta-cresol is present as a preservative at a concentration of about 3.0 mg/mL to about 4.0 mg/mL and the formulation has a pH of about 6.0 to 7.0; optionally wherein the glucagon analogue, or the pharmaceutically acceptable salt thereof, is present at a concentration of about 4 mg/mL, TRIS is present at a concentration of about 50 mM, sodium chloride is present as a tonicity modifier at a concentration of about 90 mM, meta-cresol is present as a preservative at a concentration of about 3.0 to about 4.0 mg/mL and the formulation has a pH of about 6.5;
or
b) the glucagon analogue, or the pharmaceutically acceptable salt thereof, is present at a concentration of about 3.0 to 5.0 mg/mL, TRIS, ACES or MES is present as a buffer at a concentration of about 25 mM to about 75 mM, and/or citrate, acetate or succinate is present as a buffer at a concentration of about 1 mM to about 30 mM, sodium chloride is present as a tonicity modifier at a concentration of about 50 mM to about 150 mM, meta-cresol is present as a preservative at a concentration of about 3.0 mg/mL to about 4.0 mg/mL and the formulation has a pH of about 6.0 to 7.0; optionally wherein the glucagon analogue, or the pharmaceutically acceptable salt thereof, is present at a concentration of about 4 mg/mL, TRIS, ACES, MES buffer is present at a concentration of about 50 mM or citrate, acetate and/or succinate buffer is present at a concentration of about 15 mM, sodium chloride is present as a tonicity modifier at a concentration of about 90 mM, meta-cresol is present as a preservative at a concentration of about 3.0 to about 4.0 mg/mL and the formulation has a pH of about 6.5.

11. The stable aqueous liquid formulation of any one of claims 1 to 10, wherein the buffer further comprises citrate, acetate or succinate.

12. The stable aqueous liquid formulation of any one of claims 1 to 11, wherein the citrate, acetate or succinate is present in the formulation at a concentration of 10 mM to 30 mM.

13. A delivery device comprising the liquid formulation of any one of claims 1 to 12, optionally wherein the delivery device is pre-filled syringe, an injector device, an injector pen, an adjustable dose auto-injector, a disposable auto-injector, a wearable injector, or an infusion pump.

14. A formulation of the glucagon analogue of any one of claims 1 to 12 for use in the treatment of a disease or condition selected from hypoglycaemia, type 2 diabetes, impaired glucose tolerance, type 1 diabetes, obesity, coronary heart disease, atherosclerosis, hypertension, dyslipidemia, hepatic steatosis, β-blocker poisoning, insulinoma or Von Gierkes disease.

15. The formulation of the glucagon analogue for use in the method of treatment of claim 14, wherein the disease or condition is hypoglycaemia, optionally wherein the hypoglycaemia is selected from the group consisting of: diabetic hypoglycaemia, acute insulin-induced hypoglycaemia, severe hypoglycaemia, acute hypoglycaemia, chronic hypoglycaemia, non-diabetic hypoglycaemia, reactive hypoglycaemia, fasting hypoglycaemia, drug-induced hypoglycaemia, alcohol-induced hypoglycaemia, gastric bypass-induced hypoglycaemia or hypoglycaemia occurring during pregnancy, optionally wherein the gastric bypass-induced hypoglycaemia is postprandial hypoglycaemia after Roux-en-Y gastric bypass.

16. The formulation of the glucagon analogue for use in the method of treatment of any one of claims 14 or 15, wherein the glucagon analogue is used for the treatment of a human patient.

17. A process for producing a stable aqueous liquid pharmaceutical formulation comprising a glucagon analogue which is:
Hy-HSQGTFTSDYSKYLD-Aib-ARAEEFVKWLEST-OH
or a pharmaceutically acceptable salt thereof;
the process comprising formulating (a) the glucagon analogue, or the pharmaceutically acceptable salt thereof, present at a concentration of about 0.5 mg/mL to about 10 mg/mL; (b) TRIS, ACES or MES is present as a buffer at a concentration of about 25 mM to about 75 mM, and/or citrate, acetate or succinate is present as a buffer at a concentration of about 1 mM to about 30 mM; (c) sodium chloride present as a tonicity modifier and at a concentration of about 50 mM to about 600 mM; (d) a pH of about 6.0 to about 7.0; and optionally (e) meta-cresol at a concentration of about 1.0 mg/mL to about 5.0 mg/mL to produce the stable aqueous liquid pharmaceutical formulation.

## Patentansprüche

1. Stabile, wässrige, flüssige pharmazeutische Formulierung, die ein Glucagon-Analogon, welches das folgende ist:
Hy-HSQGTFTSDYSKYLD-Aib-ARAEEFVKWLEST-OH,
oder ein pharmazeutisch annehmbares Salz davon umfasst;
wobei die Formulierung Folgendes umfasst:
(a) das Glucagon-Analogon in einer Konzentration von etwa 0,5 mg/ml bis etwa 10 mg/ml;
(b) TRIS, ACES oder MES als Puffer in einer Konzentration von etwa 25 mM bis etwa 75 mM und/oder ein Citrat, Acetat oder Succinat als Puffer in einer Konzentration von etwa 1 mM bis etwa 30 mM;
(c) Natriumchlorid als Tonizitätsmodifikator in einer Konzentration von etwa 50 mM bis etwa 600 mM; und
(d) einen pH von etwa 5,6 bis etwa 7,0.

2. Stabile, wässrige, flüssige pharmazeutische Formulierung nach Anspruch 1, wobei der TRIS-, ACES- oder MES-Puffer das Glucagon-Analogon unabhängig vom pH der Formulierung, der durch den Puffer bereitgestellt wird, direkt chemisch stabilisiert.

3. Stabile, wässrige, flüssige pharmazeutische Formulierung nach Anspruch 1 oder Anspruch 2, wobei die Formulierung:
i) für eine verbesserte chemische Stabilisierung im Vergleich zu einer Formulierung sorgt, in welcher der TRIS-, ACES- oder MES-Puffer durch einen Phosphatpuffer und/oder einen Histidinpuffer in der gleichen Konzentration und mit dem gleichen pH, der unter den gleichen Testbedingungen bestimmt wurde, ersetzt ist;
ii) ein Abbauprofil aufweist, bei dem die Formulierung nach 52 Wochen Lagerung bei 25 °C eines oder mehrere aus weniger als 5 % Pyro-Glu 4-29, weniger als 7 % Trp/Tyr-Oxidation, weniger als 4 % Kynurenin, weniger als 5 % F-4-29 + F5- 29 und/oder weniger als 2 % F3.29 nach 52 Wochen Lagerung bei 25 °C aufweist, wobei alle Prozentsätze durch HPLC bestimmt werden;
iii) ein Abbauprofil aufweist, bei dem das Glucagon-Analogon frei von Bernsteinsäure-Addition an Maleinsäure ist;
iv) im Wesentlichen kein aprotisch-polares Lösungsmittel umfasst; und/oder
v) im Wesentlichen kein Dimethylsulfoxid (DMSO) umfasst.

4. Stabile, wässrige, flüssige pharmazeutische Formulierung nach einem der vorangegangenen Ansprüche, wobei Wasser das einzige Lösungsmittel ist, das zur Herstellung der wässrigen flüssigen Formulierung verwendet wird.

5. Stabile, wässrige, flüssige pharmazeutische Formulierung nach einem der vorangegangenen Ansprüche, wobei:
i) das Glucagon-Analogon oder das pharmazeutisch annehmbare Salz davon in einer Konzentration von etwa 0,5 mg/ml, 0,6 mg/ml, 0,7 mg/ml, 1,0 mg/ml oder etwa 4,0 mg/ml vorhanden ist;
ii) der TRIS-, ACES- oder MES-Puffer als Puffer in einer Konzentration von etwa 50 mM vorhanden ist;
iii) das Natriumchlorid als Tonizitätsmodifikator in einer Konzentration von etwa 150 mM bis etwa 200 mM oder in einer Konzentration von etwa 50 mM bis etwa 150 mM vorhanden ist; und/oder
iv) die Formulierung einen pH von etwa 5,8 bis 6,7 aufweist, wobei die Formulierung gegebenenfalls einen pH von etwa 6,5 aufweist.

6. Stabile, wässrige, flüssige pharmazeutische Formulierung nach einem der vorangegangenen Ansprüche, wobei die Formulierung:
i) keinen Ionisation stabilisierenden Hilfsstoff umfasst, der aus Salzsäure, Salpetersäure, Schwefelsäure und einer Kombination davon ausgewählt ist;
ii) eine gebrauchsfertige Formulierung ist;
iii) bei 2 °C bis 8 °C zumindest 6 Monate lang, zumindest 12 Monate lang, zumindest 18 Monate lang oder zumindest 24 Monate lang stabil ist, wobei das Glucagon-Analogon in der Formulierung gegebenenfalls nach 18 Monaten Lagerung bei 2 °C bis 8 °C zumindest etwa 90 % seiner biologischen Aktivität beibehält;
iv) steril und/oder frei von Reduktionsmitteln ist;
v) zur Verabreichung an ein Individuum mittels Injektion formuliert ist, wobei die Injektion gegebenenfalls eine subkutane Injektion ist.

7. Stabile, wässrige, flüssige pharmazeutische Formulierung nach einem der vorangegangenen Ansprüche, wobei der Puffer TRIS ist.

8. Stabile, wässrige, flüssige pharmazeutische Formulierung nach einem der vorangegangenen Ansprüche, die weiters ein Konservierungsmittel umfasst, wobei das Konservierungsmittel gegebenenfalls m-Kresol, gegebenenfalls in einer Konzentration von etwa 1,0 mg/ml bis etwa 5,0 mg/ml, ist.

9. Stabile, wässrige, flüssige pharmazeutische Formulierung nach einem der Ansprüche 1 bis 6, wobei das Glucagon-Analogon oder das pharmazeutisch annehmbare Salz davon in einer Konzentration von etwa 0,75 mg/ml bis etwa 1,25 mg/ml vorhanden ist, der TRIS-, ACES- oder MES-Puffer in einer Konzentration von etwa 40 mM bis etwa 60 mM vorhanden ist, Natriumchlorid als Tonizitätsmodifikator in einer Konzentration von etwa 150 mM bis etwa 200 mM vorhanden ist und die Formulierung einen pH von etwa 6,0 bis etwa 6,8 aufweist, wobei gegebenenfalls das Glucagon-Analogon oder das pharmazeutisch annehmbare Salz davon in einer Konzentration von etwa 1,0 mg/ml vorhanden ist, TRIS in einer Konzentration von etwa 50 mM vorhanden ist, Natriumchlorid als Tonizitätsmodifikator in einer Konzentration von etwa 175 mM vorhanden ist und die Formulierung einen pH von etwa 6,5 aufweist.

10. Stabile, wässrige, flüssige pharmazeutische Formulierung nach einem der Ansprüche 1 bis 8, wobei:
a) das Glucagon-Analogon oder das pharmazeutisch annehmbare Salz davon in einer Konzentration von etwa 3,0 bis 5,0 mg/ml vorhanden ist, der TRIS-, ACES- oder MES-Puffer in einer Konzentration von etwa 40 mM bis etwa 60 mM vorhanden ist, Natriumchlorid als Tonizitätsmodifikator in einer Konzentration von etwa 50 mM bis etwa 150 mM vorhanden ist, m-Kresol als Konservierungsmittel in einer Konzentration von etwa 3,0 mg/ml bis etwa 4,0 mg/ml vorhanden ist und die Formulierung einen pH von etwa 6,0 bis 7,0 aufweist, wobei gegebenenfalls das Glucagon-Analogon oder das pharmazeutisch annehmbare Salz davon in einer Konzentration von etwa 4 mg/ml vorhanden ist, TRIS in einer Konzentration von etwa 50 mM vorhanden ist, Natriumchlorid als Tonizitätsmodifikator in einer Konzentration von etwa 90 mM vorhanden ist, m-Kresol als Konservierungsmittel in einer Konzentration von etwa 3,0 bis etwa 4,0 mg/ml vorhanden ist und die Formulierung einen pH von etwa 6,5 aufweist; oder
b) das Glucagon-Analogon oder das pharmazeutisch annehmbare Salz davon in einer Konzentration von etwa 3,0 bis 5,0 mg/ml vorhanden ist, der TRIS-, ACES- oder MES-Puffer in einer Konzentration von etwa 25 mM bis etwa 75 mM vorhanden ist und/oder Citrat, Acetat oder Succinat als Puffer in einer Konzentration von etwa 1 mM bis etwa 30 mM vorhanden ist, Natriumchlorid als Tonizitätsmodifikator in einer Konzentration von etwa 50 mM bis etwa 150 mM vorhanden ist, m-Kresol als Konservierungsmittel in einer Konzentration von etwa 3,0 mg/ml bis etwa 4,0 mg/ml vorhanden ist und die Formulierung einen pH von etwa 6,0 bis 7,0 aufweist, wobei gegebenenfalls das Glucagon-Analogon oder das pharmazeutisch annehmbare Salz davon in einer Konzentration von etwa 4 mg/ml vorhanden ist, der TRIS-, ACES-, MES-Puffer in einer Konzentration von etwa 50 mM vorhanden ist oder ein Citrat-, Acetat- und/oder Succinat-Puffer in einer Konzentration von etwa 15 mM vorhanden ist, Natriumchlorid als Tonizitätsmodifikator in einer Konzentration von etwa 90 mM vorhanden ist, m-Kresol als Konservierungsmittel in einer Konzentration von etwa 3,0 bis etwa 4,0 mg/ml vorhanden ist und die Formulierung einen pH von etwa 6,5 aufweist.

11. Stabile, wässrige, flüssige pharmazeutische Formulierung nach einem der Ansprüche 1 bis 10, wobei der Puffer weiters Citrat, Acetat oder Succinat umfasst.

12. Stabile, wässrige, flüssige pharmazeutische Formulierung nach einem der Ansprüche 1 bis 11, wobei das Citrat, Acetat oder Succinat in der Formulierung in einer Konzentration von 10 mM bis 30 mM vorhanden ist.

13. Abgabevorrichtung, die eine stabile, wässrige, flüssige pharmazeutische Formulierung nach einem der Ansprüche 1 bis 12 umfasst, wobei die Abgabevorrichtung gegebenenfalls eine vorbefüllte Spritze, eine Injektionsvorrichtung, ein Injektorstift, ein Autoinjektor mit einstellbarer Dosis, ein Einweg-Autoinjektor, eine tragbare Injektionsvorrichtung oder eine Infusionspumpe ist.

14. Formulierung eines Glucagon-Analogons nach einem der Ansprüche 1 bis 12 zur Verwendung bei der Behandlung einer Erkrankung oder eines Leidens, die aus Hypoglykämie, Diabetes vom Typ 2, gestörter Glucosetoleranz, Diabetes vom Typ 1, Adipositas, einer koronaren Herzerkrankung, Atherosklerose, Hypertonie, Dyslipidämie, hepatischer Steatose, einer β-Blocker-Vergiftung, Insulinomen oder der Von-Gierkes-Krankheit ausgewählt ist.

15. Formulierung eines Glucagon-Analogons zur Verwendung in einem Behandlungsverfahren nach Anspruch 14, wobei die Erkrankung oder das Leiden Hypoglykämie ist, wobei die Hypoglykämie gegebenenfalls aus der aus Folgendem bestehenden Gruppe ausgewählt ist: diabetischer Hypoglykämie, akuter insulininduzierter Hypoglykämie, schwerer Hypoglykämie, akuter Hypoglykämie, chronischer Hypoglykämie, nichtdiabetischer Hypoglykämie, reaktiver Hypoglykämie, Hypoglykämie bei Nahrungskarenz, arzneimittelinduzierter Hypoglykämie, alkoholinduzierter Hypoglykämie, durch einen Magenbypass induzierter Hypoglykämie oder Hypoglykämie während der Schwangerschaft, wobei die durch einen Magenbypass induzierte Hypoglykämie gegebenenfalls postprandiale Hypoglykämie nach einem Roux-en-Y-Magenbypass ist.

16. Formulierung eines Glucagon-Analogons zur Verwendung in einem Behandlungsverfahren nach einem der Ansprüche 14 oder 15, wobei das Glucagon-Analogon zur Behandlung eines menschlichen Patienten verwendet wird.

17. Verfahren zur Herstellung einer stabilen, wässrigen, flüssigen pharmazeutischen Formulierung, die ein Glucagon-Analogon, welches das folgende ist:
Hy-HSQGTFTSDYSKYLD-Aib-ARAEEFVKWLEST-OH,
oder ein pharmazeutisch annehmbares Salz davon umfasst;
wobei das Verfahren das Formulieren von (a) dem Glucagon-Analogon oder dem pharmazeutisch annehmbaren Salz davon, das in einer Konzentration von etwa 0,5 mg/ml bis etwa 10 mg/ml vorhanden ist; (b) TRIS, ACES oder MES als Puffer in einer Konzentration von etwa 25 mM bis etwa 75 mM und/oder Citrat, Acetat oder Succinat als Puffer in einer Konzentration von etwa 1 mM bis etwa 30 mM; (c) Natriumchlorid als Tonizitätsmodifikator in einer Konzentration von etwa 50 mM bis etwa 600 mM; (d) einen pH von etwa 6,0 bis etwa 7,0; und gegebenenfalls (e) m-Kresol in einer Konzentration von etwa 1,0 mg/ml bis etwa 5,0 mg/ml; umfasst, um die stabile, wässrige, flüssige pharmazeutische Formulierung herzustellen.

## Revendications

1. Formulation pharmaceutique liquide aqueuse stable comprenant un analogue du glucagon, qui est :
Hy-HSQGTFTSDYSKYLD-Aib-ARAEEFVKWLEST-OH
ou un sel de celui-ci pharmaceutiquement acceptable ;
où la formulation comprend :
(a) l'analogue du glucagon présent à une concentration d'environ 0,5 mg/ml à environ 10 mg/ml ;
(b) du TRIS, ACES ou MES présent en tant que tampon à une concentration d'environ 25 mM à environ 75 mM, et/ou du citrate, de l'acétate ou du succinate présent en tant que tampon à une concentration d'environ 1 mM à environ 30 mM ;
(c) du chlorure de sodium présent en tant que modificateur de tonicité et à une concentration d'environ 50 mM à environ 600 mM ; et
(d) un pH d'environ 5,6 à environ 7,0.

2. Formulation liquide aqueuse stable selon la revendication 1, dans laquelle le tampon TRIS, ACES ou MES stabilise chimiquement et directement l'analogue du glucagon indépendant du pH de la formulation fourni par le tampon.

3. Formulation liquide aqueuse stable selon la revendication 1 ou la revendication 2, dans laquelle la formulation :
i) offre une stabilisation chimique améliorée par rapport à une formulation dans laquelle le tampon TRIS, ACES ou MES est remplacé par un tampon phosphate et/ou un tampon histidine de même concentration et pH évalué dans les mêmes conditions de test ;
ii) possède un profil de dégradation après 52 semaines de stockage à 25 °C selon lequel la formulation contient un ou plusieurs de moins de 5 % de Pyro-Glu 4-29, moins de 7 % d'oxydation de Trp/Tyr, moins de 4 % de kynurénine, moins de 5 % de F-4-29 + F5-29 et/ou moins de 2 % de F3.29 après un stockage pendant 52 semaines à 25 °C, où tous les pourcentages sont déterminés par HPLC ;
iii) possède un profil de dégradation dans lequel l'analogue du glucagon est dépourvu d'addition d'acide succinique à l'acide maléique ;
iv) ne comprend essentiellement pas de solvant polaire aprotique ; et/ou
v) ne comprend essentiellement pas de diméthylsulfoxyde (DMSO).

4. Formulation liquide aqueuse stable selon l'une quelconque des revendications précédentes, dans laquelle l'eau est le seul solvant utilisé pour fabriquer la formulation liquide aqueuse.

5. Formulation liquide aqueuse stable selon l'une quelconque des revendications précédentes, dans laquelle :
i) l'analogue du glucagon, ou le sel de celui-ci pharmaceutiquement acceptable, est présent à une concentration d'environ 0,5 mg/ml, 0,6 mg/ml, 0,7 mg/ml, 1,0 mg/ml ou environ 4,0 mg/ml ;
ii)le tampon TRIS, ACES ou MES est présent en tant que tampon à une concentration d'environ 50 mM ;
iii) le chlorure de sodium est présent en tant que modificateur de tonicité à une concentration d'environ 150 mM à environ 200 mM ou à une concentration d'environ 50 mM à environ 150 mM ; et/ou
iv) la formulation possède un pH d'environ 5,8 à 6,7, facultativement où la formulation possède un pH d'environ 6,5.

6. Formulation liquide aqueuse stable selon l'une quelconque des revendications précédentes, où la formulation :
i) ne comprend pas d'excipient stabilisant l'ionisation sélectionné parmi l'acide chlorhydrique, l'acide nitrique, l'acide sulfurique ou une combinaison de ceux-ci ;
ii) est une formulation prête à l'emploi ;
iii) est stable à 2-8 °C pendant au moins 6 mois, au moins 12 mois, au moins 18 mois ou au moins 24 mois, facultativement dans laquelle l'analogue du glucagon dans la formulation conserve au moins environ 90 % de son activité biologique après 18 mois de stockage à 2-8 °C ;
iv) est stérile et/ou dépourvue d'agent réducteur ;
v) est formulée pour une administration à un sujet par injection, facultativement dans laquelle l'injection est une injection sous-cutanée.

7. Formulation liquide aqueuse stable selon l'une quelconque des revendications précédentes, dans laquelle le tampon est du TRIS.

8. Formulation liquide aqueuse stable selon l'une quelconque des revendications précédentes, comprenant en outre un conservateur, facultativement dans laquelle le conservateur est le métacrésol, facultativement à une concentration d'environ 1,0 mg/ml à environ 5,0 mg/ml.

9. Formulation liquide aqueuse stable selon l'une quelconque des revendications 1 à 6, dans laquelle l'analogue du glucagon, ou le sel de celui-ci pharmaceutiquement acceptable, est présent à une concentration d'environ 0,75 mg/ml à environ 1,25 mg/ml, le tampon TRIS, ACES ou MES est présent à une concentration d'environ 40 mM à environ 60 mM, le chlorure de sodium est présent en tant que modificateur de tonicité à une concentration d'environ 150 mM à environ 200 mM et la formulation possède un pH d'environ 6,0 à environ 6,8, facultativement dans laquelle l'analogue du glucagon, ou le sel de celui-ci pharmaceutiquement acceptable, est présent à une concentration d'environ 1,0 mg/ml, le TRIS est présent à une concentration d'environ 50 mM, le chlorure de sodium est présent en tant que modificateur de tonicité à une concentration d'environ 175 mM et la formulation possède un pH d'environ 6,5.

10. Formulation liquide aqueuse stable selon l'une quelconque des revendications 1 à 8, dans laquelle :
a) l'analogue du glucagon, ou le sel de celui-ci pharmaceutiquement acceptable, est présent à une concentration d'environ 3,0 à 5,0 mg/ml, le tampon TRIS, ACES ou MES est présent à une concentration d'environ 40 mM à environ 60 mM, le chlorure de sodium est présent en tant que modificateur de tonicité à une concentration d'environ 50 mM à environ 150 mM, le métacrésol est présent en tant que conservateur à une concentration d'environ 3,0 mg/ml à environ 4,0 mg/ml et la formulation possède un pH d'environ 6,0 à 7,0 ; facultativement dans laquelle l'analogue du glucagon, ou le sel de celui-ci pharmaceutiquement acceptable, est présent à une concentration d'environ 4 mg/ml, le TRIS est présent à une concentration d'environ 50 mM, le chlorure de sodium est présent en tant que modificateur de tonicité à une concentration d'environ 90 mM, le métacrésol est présent en tant que conservateur à une concentration d'environ 3,0 à environ 4,0 mg/ml et la formulation possède un pH d'environ 6,5 ;
ou
b) l'analogue du glucagon, ou le sel de celui-ci pharmaceutiquement acceptable, est présent à une concentration d'environ 3,0 à 5,0 mg/ml, le TRIS, ACES ou MES est présent en tant que tampon à une concentration d'environ 25 mM à environ 75 mM, et/ou le citrate, l'acétate ou le succinate est présent en tant que tampon à une concentration d'environ 1 mM à environ 30 mM, le chlorure de sodium est présent en tant que modificateur de tonicité à une concentration d'environ 50 mM à environ 150 mM, le métacrésol est présent en tant que conservateur à une concentration d'environ 3,0 mg/ml à environ 4,0 mg/ml et la formulation possède un pH d'environ 6,0 à 7,0 ; facultativement dans laquelle l'analogue du glucagon, ou le sel de celui-ci pharmaceutiquement acceptable, est présent à une concentration d'environ 4 mg/ml, le tampon TRIS, ACES, MES est présent à une concentration d'environ 50 mM ou le tampon citrate, acétate et/ou succinate est présent à une concentration d'environ 15 mM, le chlorure de sodium est présent en tant que modificateur de tonicité à une concentration d'environ 90 mM, le métacrésol est présent en tant que conservateur à une concentration d'environ 3,0 à environ 4,0 mg/ml et la formulation possède un pH d'environ 6,5.

11. Formulation liquide aqueuse stable selon l'une quelconque des revendications 1 à 10, dans laquelle le tampon comprend en outre du citrate, de l'acétate ou du succinate.

12. Formulation liquide aqueuse stable selon l'une quelconque des revendications 1 à 11, dans laquelle le citrate, l'acétate ou le succinate est présent dans la formulation à une concentration de 10 mM à 30 mM.

13. Dispositif de délivrance comprenant la formulation liquide selon l'une quelconque des revendications 1 à 12, facultativement dans lequel le dispositif de délivrance est une seringue préremplie, un dispositif injecteur, un stylo injecteur, un auto-injecteur à dose ajustable, un auto-injecteur jetable, un injecteur portable, ou une pompe à perfusion.

14. Formulation de l'analogue du glucagon selon l'une quelconque des revendications 1 à 12 destinée à être utilisée dans le traitement d'une maladie ou d'une affection sélectionnée parmi l'hypoglycémie, le diabète de type 2, l'intolérance au glucose, le diabète de type 1, l'obésité, une coronaropathie, l'athérosclérose, l'hypertension, la dyslipidémie, la stéatose hépatique, une intoxication par les β-bloquants, un insulinome ou la maladie de Von Gierke.

15. Formulation de l'analogue du glucagon destinée à être utilisée dans le procédé de traitement selon la revendication 14, dans laquelle la maladie ou l'affection est l'hypoglycémie, facultativement dans laquelle l'hypoglycémie est sélectionnée dans le groupe consistant en : l'hypoglycémie diabétique, l'hypoglycémie aiguë induite par l'insuline, l'hypoglycémie sévère, l'hypoglycémie aiguë, l'hypoglycémie chronique, l'hypoglycémie non diabétique, l'hypoglycémie réactive, l'hypoglycémie à jeun, l'hypoglycémie induite par les médicaments, l'hypoglycémie induite par l'alcool, l'hypoglycémie induite par un pontage gastrique ou l'hypoglycémie survenant pendant la grossesse, facultativement dans laquelle l'hypoglycémie induite par un pontage gastrique est l'hypoglycémie post-prandiale après un pontage gastrique de Roux-en-Y.

16. Formulation de l'analogue du glucagon destinée à être utilisée dans le procédé de traitement selon l'une quelconque des revendications 14 ou 15, dans laquelle l'analogue du glucagon est utilisé pour le traitement d'un patient humain.

17. Procédé de production d'une formulation pharmaceutique liquide aqueuse stable comprenant un analogue du glucagon qui est :
Hy-HSQGTFTSDYSKYLD-Aib-ARAEEFVKWLEST-OH
ou un sel de celui-ci pharmaceutiquement acceptable ;
le procédé comprenant la formulation de (a) l'analogue du glucagon, ou du sel de celui-ci pharmaceutiquement acceptable, présent à une concentration d'environ 0,5 mg/ml à environ 10 mg/ml ; (b) TRIS, ACES ou MES présent en tant que tampon à une concentration d'environ 25 mM à environ 75 mM, et/ou citrate, de l'acétate ou du succinate présent en tant que tampon à une concentration d'environ 1 mM à environ 30 mM ; (c) chlorure de sodium présent en tant que modificateur de tonicité à une concentration d'environ 50 mM à environ 600 mM ; (d) un pH d'environ 6,0 à environ 7,0 ; et facultativement (e) métacrésol à une concentration d'environ 1,0 mg/ml à environ 5,0 mg/ml afin de produire la formulation pharmaceutique liquide aqueuse stable.
